# EUROPEAN PATENT SPECIFICATION

(11) **EP 2 016 414 B1**
(45) Date of publication and mention of the grant of the patent: **02.09.2015**
(21) Application number: 07797347.7
(22) Date of filing: 04.05.2007
(51) Int. Cl.: A61K 39/00, C12N 5/0783, G01N 33/50

(54) **T-CELL VACCINE**
T-ZELL VAKZIN
VACCIN À LYMPHOCYTES T

(30) Priority: 05.05.2006 US 746611 P; 22.05.2006 US 747903 P
(43) Date of publication of application: 21.01.2009
(60) Divisional application: 11188676.8
(73) Proprietor: Opexa Therapeutics, The Woodlands TX 77381 (US)
(72) Inventor: WILLIAMS, Jim, C., The Woodlands, TX 77382 (US); MONTGOMERY, Mitzi, M., Jay, OK 74346-0527 (US); NEWSOM, Brian, S., Spring, TX 77379 (US)
(74) Representative: Walton, Seán Malcolm
(86) International application number: PCT/US2007/068304
(87) International publication number: WO 2007/131210

(56) References cited:
- MEDAER R ET AL: "Depletion of myelin-basic-protein autoreactive T cells by T-cell vaccination: pilot trial in multiple sclerosis" LANCET THE, LANCET LIMITED. LONDON, GB, vol. 346, no. 8973, 23 September 1995 (1995-09-23), pages 807-808, XP002966968 ISSN: 0140-6736
- ACHIRON A ET AL: "T cell vaccination in multiple sclerosis relapsing-remitting nonresponders patients" CLINICAL IMMUNOLOGY, ACADEMIC PRESS, US, vol. 113, no. 2, 1 November 2004 (2004-11-01), pages 155-160, XP004576819 ISSN: 1521-6616
- VAN DER AA A ET AL: "T cell vaccination in multiple sclerosis patients with autologous CSF-derived activated T cells: results from a pilot study." CLINICAL AND EXPERIMENTAL IMMUNOLOGY JAN 2003, vol. 131, no. 1, January 2003 (2003-01), pages 155-168, XP002533299 ISSN: 0009-9104
- ZHANG JINGWU Z ET AL: "T cell vaccination in multiple sclerosis: Results of a preliminary study" 1 February 2002 (2002-02-01), JOURNAL OF NEUROLOGY - ZEITSCHRIFT FUER NEUROLOGIE, SPRINGER VERLAG, BERLIN, DE, PAGE(S) 212 - 218 , XP002292820 ISSN: 0340-5354 * the whole document *
- OTA K ET AL: "T-CELL RECOGNITION OF AN IMMUNODOMINANT MYELIN BASIC PROTEIN EPITOPE IN MULTIPLE SCLEROSIS" NATURE, NATURE PUBLISHING GROUP, LONDON, UK, vol. 346, no. 6280, 12 July 1990 (1990-07-12), pages 183-187, XP000652362 ISSN: 0028-0836
- ZHANG J. ET AL.: 'MHC-Restricted Depletion of Human Myelin Basic Protein-Reactive T cells by T cell Vaccination' SCIENCE vol. 261, 1993, pages 1451 - 1454, XP002091570
- VERGELLI M.: 'Short-Term Evolution of Autoreactive T cell Repertoire in Multiple Sclerosis' J. NEUROSCI. RES. vol. 66, 2001, pages 517 - 524
- ZHANG J. ET AL: 'MHC-restricted depletion of human myelin basic protein-reactive T cells by T cell vaccination' SCIENCE, AMERICAN ASSOCIATION FOR THE ADVANCEMENT OF SCIENCE, WASHINGTON, DC; US vol. 261, no. 5127, 10 September 1993, pages 1451 - 1454, XP002091570 ISSN: 0036-8075
- M. VERGELLI ET AL: 'Short-term evolution of autoreactive T cell repertoire in multiple sclerosis' JOURNAL OF NEUROSCIENCE RESEARCH vol. 66, no. 3, 01 November 2001, pages 517 - 524, XP055006174 DOI: 10.1002/jnr.1243 ISSN: 0360-4012
- [Online] Retrieved from the Internet: <URL:http://www.equities.com/research/cryst al-research/pdfs/PFTR.OB_EIO_11-21-05.pdf>
- MEINI E. ET AL: 'MYELIN BASIC PROTEIN-SPECIFIC T LYMPHOCYTE REPERTOIRE IN MULTIPLE SCLEROSIS COMPLEXITY OF THE RESPONSE AND DOMINANCE OF NESTED EPITOPES DUE TO RECRUITMENT OF MULTIPLE T CELL CLONES' JOURNAL OF CLINICAL INVESTIGATION, AMERICAN SOCIETY FOR CLINICAL INVESTIGATION, US vol. 92, no. 6, 01 January 1993, pages 2633 - 2643, XP001010559 DOI: 10.1172/JCI116879 ISSN: 0021-9738
- ANONYMOUS: 'PharmaFrontiers presents positive Tovaxin(TM) research at international multiple sclerosis meeting', [Online] November 2005, pages 1 - 3 Retrieved from the Internet: <URL:http://www.msmusings.com/archive70/74/ FYI,%20two%20articles%20tovaxin.htm> [retrieved on 2013-05-08]

## Description

### FIELD OF THE INVENTION

The present invention relates to T-cell vaccines and methods of preparing these vaccines. The T-cell vaccines may be used to treat autoimmune diseases, such as multiple sclerosis.

### BACKGROUND OF THE INVENTION

Multiple sclerosis (MS) is an inflammatory disease characterized by the destruction of myelin in the central nervous system (CNS). Growing evidence implicates autoimmune T cell responses to myelin antigens such as myelin basic protein (MBP), myelin oligodendrocyte glycoprotein (MOG) and myelin proteolipid protein (PLP) in the pathogenesis of the disease (Stinissen et al., Crit. Rev. Immunol. 1997; 17:33-75). Activation and clonal expansion of myelin-reactive T cells, followed by their entry into the CNS through the blood brain barrier, results in injury to the myelin membrane. MBP-reactive T cells are found to undergo *in vivo* activation and occur at high precursor frequency in the blood and cerebrospinal fluid of patients with MS (Zhang et al., J. Exp. Med., 1994; 179:973-984; Chou et al., J. Neuroimmunol., 1992; 38:105-114: Allegretta et al., Science, 1990; 247:718-721). Recent strategies for combating the disease have focused on diminishing the number of activated myelin-reactive T cells by a procedure termed T cell vaccination. Repeated inoculations with MBP-reactive T cells that have been inactivated by chemical treatment or irradiation has been demonstrated to prevent or cure experimental autoimmune encephalomyelitis (EAE), an animal model for MS (Ben-Nun et al., Eur. J. Immunol., 1981; 11:195-204).

T cell vaccination has been advanced to clinical trials in patients with MS. In a pilot clinical trial, three subcutaneous inoculations with irradiated MBP-reactive T cells induced T cell responses that reduced the number of circulating MBP-reactive T cells to undetectable levels in a number of subjects (Zhang et al., Science, 1993; 261:1451-1454, Medear et al., Lancet 1995; 346:807-808). Consequently, patients who were administered the vaccine demonstrated clinical improvement evidenced as a reduction in rate of relapse and MRI lesion activity. The safety profile and technological feasibility of the pilot clinical trial were excellent. Patients had improvements in subjective physical and psychological parameters, and the Kurtzke Expanded Disability Status Scale (EDSS), which is an objective measure of a patient's physical disability.

The initial T cell vaccination strategy used full length MBP to identify autoreactive epitopes. Vaccination with inactivated autoreactive T cells produced using this method was initially believed to be sufficient to treat MS. However, subsequent studies revealed that some time after vaccination, myelin antigen-reactive T cells reappeared. (Zhang *et al*.). These myelin antigen-reactive T cells frequently exhibited a different reactivity profile than those initially identified, a process termed epitope-shifting. The epitopes recognized by the reappearing T cells were often cryptic epitopes, not accessible to the immune system in the full-length polypeptide and represented a shift in immunodominance.

Subsequent T cell vaccination strategies have relied on the use of immunodominant myelin antigen epitopes to identify autoreactive T cells of a given MS patient. The structural features of TCR-MHC class II and/or I -peptide interactions drive the T cell response directed towards prominent epitopes. While the molecular basis for immunodominance is related to peptide affinity to MHC, T cells recognize a complex of antigenic fragments associated with MHC on antigen-presenting cells (APC). Immunodominance is considered to be a higher relative number (or frequency) of antigen-specific T cells that reach a threshold activation (e.g., stimulation index) response for a particular antigen. Immunodominance is variably used to describe either the most frequently detectable response among tested individuals or the strongest response within a single individual, yet factors determining either inter- or intra-individual immunodominance are still poorly understood.

Immunodominance is a central feature of T-cell responses to antigens. Of the many peptides present in complex antigens, responses to peptides can be ordered based on the numbers and/or activity of responding T cells into a relatively stable hierarchy. Despite its importance to understanding immune responses and designing vaccines, immunodominance is poorly understood at the mechanistic level. Immunodominance is not simply explained by the numbers of peptide complexes generated by antigen-presenting cells (APCs), the affinities of peptides for class I or class II molecules, or the affinities of T-cell receptors for peptide-class I or class II complexes, though each of these parameters contributes to the phenomenon.

Using immunodominant epitopes in production of a T-cell vaccine was based on the recognition that although T cell responses to myelin antigens are heterogeneous in the epitope recognition among different individuals, certain regions of myelin antigens, such as residues 83-99 and 151-170 of MBP, are preferentially recognized in some patients with MS. (Ota et al., Nature, 1990; 346:183-187). Autologous T cells reactive to the immunodominant peptides were expanded and inactivated, then injected into the patient from which they were isolated. Although this strategy led to a decrease in rates of disease progression, the disease course of the patients continues to progress. Thus, there exists a need for improved T-cell vaccines.

Achiron et al., Clin. Immunology, 2004, disclose the immunization of multiple sclerosis patients with autologous attenuated T cell lines that were activated with synthetic peptides of MBP (amino acids 83-99, 87-110 and 151-170) and MOG (amino acids 6-26 and 34-56). The T cell vaccination showed beneficial clinical effects in the vaccinated patients.

The press release of PharmaFrontiers presents Tovaxin, which is a trivalent formulation of attenuated peptide-reactive T cells against MBP, PLP and MOG. Two peptides of each MBP, PLP and MOG were used for the production of the vaccine. Tovaxin treatment depleted myelin-peptide reactive T cells in patients with multiple sclerosis.
(http://www.msmusings.com/archive70/74/FYI,%20two%20articles%20tovaxin.htm)

### SUMMARY OF THE INVENTION

The present invention is as set out in the claims.

A method of identifying an epitope within a polypeptide antigen for an autoreactive T-cell is disclosed. A sample comprising T cells isolated from a host may be provided. One or more different peptides may be added to a plurality of portions of the sample. The sequences of the peptides may collectively comprise a portion of the sequence of the polypeptide antigen. A portion of the sample comprising activated autoreactive T cells may be identified. A peptide that activates the autoreactive T cells may comprise the epitope.

The sequences of the peptides collectively comprise the complete sequence of the polypeptide antigen. The polypeptide antigen may be MBP, PLP, MOG or a combination thereof. The different peptides comprise overlapping sequence of 4-19 amino acids. The different peptides are 8-20 amino acids. The number of stimulated autoreactive T cells may be increased by at least a factor of about 2 to 4 compared to a control.

A method of preparing a T cell vaccine as set out in claim 1 is provided. A sample comprising T cells isolated from a patient is provided. The T cells are contacted with one or more different peptide mixes which activate autoreactive T cells. The activated autoreactive T cells are expanded. The autoreactive T cells are then be attenuated. The different peptides stimulate autoreactive T-cells with a stimulation index above a predetermined value. The antigenic polypeptide is MBP, PLP and MOG.

A method of detecting epitope shift in a T cell mediated disease is also disclosed, The epitopes of an autoreactive antigen may be identified, as described above. The epitopes may be compared to a control. Epitope shift may have occurred if the epitopes are different. Detecting epitope shift may be used to diagnose epitope shift in a subject. Detecting epitope shift may also be used to monitor epitope shift in a subject by comparing epitopes to epitopes at a previous time.

A T cell vaccine is also disclosed. The vaccine comprise T cells that are specific to an antigenic polypeptide. The vaccine comprises T cells that recognize each epitope of the antigenic polypeptides capable of producing a stimulation index above a predetermined value. The antigenic polypeptides are MBP, PLP, and MOG. The vaccine may comprise less than 50% T cells that recognize the epitopes of the antigenic polypeptide capable of producing a stimulation index of less than a predetermined value. The T cells may comprise cell markers including, but not limited to, CD3, CD4, CD8, CD25, TCR αβ, TCR γδ, HSP60 (heat-shock protein 60) or a combination thereof.

### BRIEF DESCRIPTION OF THE DRAWINGS

Figure 1 shows EAA analysis of reactivity of one patient's T cells to MOG peptide mixes.
Figure 2 shows the frequencies of reactivity to MBP peptide mixes among T cells from 48 subjects.
Figure 3 shows the frequencies of reactivity to PLP peptide mixes among T cells from 48 subjects.
Figure 4 shows the frequencies of reactivity to MOG peptide mixes among T cells from 48 subjects.
Figure 5 shows the ProPred binding predictions within MOG protein for the 3 HLA alleles of the patient. The red amino acid residues represent predicted anchor residues for binding within the HLA groove, while the yellow residues represent the other residues that would fit within the groove.
Figure 6 shows EAA analysis of reactivity of one patient's T cells to MBP peptide mixes.
Figure 7 shows the percentage expression of TCR V beta chains at baseline and after 18 days of stimulation with MBPm10 peptides using T cells from one patient.
Figure 8 shows EAA analysis of reactivity of one patient's T cells to myelin peptide mixes.
Figure 9, shows growth of T cells that exhibited strong reactivity to two myelin peptides by EAA analysis as shown in Figure 8.
Figure 10 shows TCR V beta focusing that occurred in one patient's T cell line as the culture progressed from Day 9 to Day 16 while being stimulated by the peptide mix PLPm27.
Figure 11 shows some of the myelin-reactive T cell frequency data from a patient that had undergone a first retreatment series of three myelin-reactive T cell vaccines.
Figure 12 shows the reactivity of a patient's Week 24 T cells to peptides spanning full length MBP. Boxes around bars indicate the peptides that contain the two immunodominant MBP sequences previously used in the T cell frequency analysis (TCFA).
Figure 13 shows the reactivity of a patients Week 24 T cells to peptides spanning full length PLP. Boxes around bars indicate the peptides that contain the two immunodominant PLP sequence previously used in the TCFA.
Figure 14 shows the reactivity of a patients Week 24 T cells to peptides spanning full length MOG. Boxes around bars indicate the peptides that contain the two immunodominant MOG sequences previously used in the TCFA.
Figure 15 shows the reactivity pattern to MBP peptides in eight patients. The horizontal red line shows the SI cut-off of 3. Boxes around bars indicate the immunodominant peptides that were previously used.
Figure 16 shows the reactivity pattern to PLP peptides in eight patients. The horizontal red line shows the SI cut-off of 3. Boxes around bars indicate the immunodominant peptides that were previously used.
Figure 17 shows the reactivity pattern to MOG peptides in eight patients. The horizontal red line shows the SI cut-off of 3. Boxes around bars indicate the immunodominant peptides that were previously used.
Figure 18 shows the mean SIs against MBP for samples from a total of 15 MS patients tested using the EAA. Boxes around bars indicate immunodominant peptides.
Figure 19 shows that mean SIs against PLP for samples from a total of 15 MS patients tested using the EAA. Boxes around bars indicate immunodominant peptides.
Figure 20 shows that mean SIs against MOG for samples from a total of 15 MS patients tested using the EAA. Boxes around bars indicate immunodominant peptides.
Figure 21 shows the frequency of reactivity to MBP peptides among 54 subjects. The subjects were healthy ("Norm"), had only blood drawn ("MD"), were enrolled in a repeat vaccination study ("Ext"), or were enrolled in a dose escalation study ("DES"). Boxes around bars indicate locations of the immunodominant peptides used in producing the vaccine of Example 1.
Figure 22 shows the frequency of reactivity to PLP peptides among 54 subjects. The subjects were healthy ("Norm"), had only blood drawn ("MD"), were enrolled in a repeat vaccination study ("Ext"), or were enrolled in a dose escalation study ("DES"). Boxes around bars indicate locations of the immunodominant peptides used in producing the vaccine of Example 1.
Figure 23 shows the frequency of reactivity to MBP peptides among 54 subjects. The subjects were healthy ("Norm"), had only blood drawn ("MD"), were enrolled in a repeat vaccination study ("Ext"), or were enrolled in a dose escalation study ("DES"). Boxes around bars indicate locations of the immunodominant peptides used in producing the vaccine of Example 1.
Figure 24 shows growth curves of five myelin-reactive T cells that had exhibited a stimulation index of less than 2.0. The T cells were isolated from two patients.
Figures 25A-H show a map of 429 assay (unique assays in rows and peptides in columns). Assays for each patient are listed in order of date performed. Positive peptide mixes are shown in grey.
Figure 26 shows a map of 65 assay (unique assays in rows and peptides in columns). Assays for each patient are listed in order of date performed. Positive peptide mixes are shown in grey.
Figure 27 shows epitope shift over time in four subjects.

### DETAILED DESCRIPTION

A T cell vaccine isdisclosed comprising T cells specific for epitopes of an antigenic polypeptide. Alsodisclosed are methods of identifying such epitopes and methods of preparing such a vaccine. The vaccine may be a personalized vaccine. Other aspects will become apparent to the skilled artisan by the following description.

### 1. Definitions.

The terminology used herein is for the purpose of describing particular embodiments only and is not intended to be limiting. As used in the specification and the appended claims, the singular forms "a," "an" and "the" include plural referents unless the context clearly dictates otherwise.

"Peptide" or "polypeptide" may mean a linked sequence of amino acids and may be natural, synthetic, or a modification or combination of natural and synthetic amino acids.

"Treatment" or "treating," when referring to protection of an animal from a disease, may mean preventing, suppressing, repressing, or completely eliminating the disease. Preventing the disease involves administering a composition of the present invention to an animal prior to onset of the disease. Suppressing the disease involves administering a composition of the present invention to an animal after induction of the disease but before its clinical appearance. Repressing the disease involves administering a composition of the present invention to an animal after clinical appearance of the disease.

### a. substantially identical

"Substantially identical" used herein may mean that a first and second sequence are at least 60%, 65%, 70%, 75%, 80%, 85%, 90%, 95%, 97%, 98% or 99% identical over a region of 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95, 100 or more amino acids.

### 2. T-Cell Immune Response

In order to initiate a specific immune response to a myelin-reactive T-cell (MRTC), the immune system must be able to identify the autoreactive T cells involved in pathogenesis and isolate particular protein products that will hone the efforts of host defense. Implicit to this model of counteraction is the processing of an immunogenic peptide epitope and its presentation on the surface of antigen-presenting cells. The result of these actions is the induction of a T-cell response that recruits and engages the other molecular participants of the immune response. At the core of this immune system element is the Major Histocompatibility Complex (MHC). Located on human chromosome 6, the MHC is a highly polymorphic set of genes that encode for molecules essential to self / non-self discrimination and antigen processing and presentation. The power of this multigenic complex lies in its polymorphism, which enables different allelic class I and class II products to bind an almost infinite array of peptides. The nature of the MHC suggests the now fundamental concept of self-MHC restriction. CD4+ T cells are activated only by antigen presenting cells that share class II MHC alleles with them; that is, antigen recognition by CD4+ helper T (Th) cells is class II MHC restricted. Antigen recognition by CD8+ cytotoxic T (Tc) cells, on the other hand, is class I MHC restricted.

A central dogma of the T cell immune response is the presentation of the peptide by the human leukocyte antigen (HLA, also known as the MHC). HLA molecules are present on the surface of antigen presenting cells (APC). These HLA molecules (of which there are 100's of different alleles) make up the HLA phenotype of a patient. A peptide to be presented to a T-cell must bind specifically within the MHC I or II groove created by the HLA molecule.

As discussed above, T-cell vaccines produced using full-length antigenic proteins were unsuccessful. Full length antigens are dependent upon processing by the APC in order for the proper peptide to be presented by the HLA. Incomplete processing decreases the ability of the HLA to present disease-relevant epitopes.

The initial attempt to overcome the problem of processing the full-length antigen was to instead produce the vaccine using peptides. In order to determine which peptides were the appropriate stimulatory antigen, screens were performed of MS patients to identify immunodominant epitopes. These immunodominant epitopes cover in reality a still minor portion of the entire reactivity among individuals with MS. Provided herein is a method to detect the truly individually specific immunoreactive (disease-relevant) peptides within each individual. The method can also be used to trace these idiotypic cells. The method may also be used to detect new pathogenic idiotypes as they arise and monitor for persistence of the suppression of others.

Even though there may be additional disease-relevant peptides for a given patient, the use of immunodominant epitopes was considered to be sufficient because vaccination of a patient with such a vaccine would lead to an anti-idiotypic and anti-ergotypic immune response. The anti-idiotypic response was believed to be directed to the specific population of T-cells in the vaccine, whereas the anti-ergotypic response was believed to be directed to all activated T-cell populations.

Using the same set of peptides for every patient does not give any consideration to the individual's HLA phenotype and therefore peptide binding to their APCs is not optimized. The use of only immunodominant peptides frequently results in poorly growing cultures as the T cells are not optimally stimulated by such peptides. For HLA alleles that are known, the production of a T-cell vaccine using peptides that are capable of binding to the patient's HLA generally leads to the production of a robust vaccine. For those unknown HLA alleles, however, the peptides that bind cannot be predicted and therefore must be used in a scanning assay to produce a robust response. Moreover, even for known alleles, the prediction model is not 100% accurate. Therefore, the EAA approach described herein may also limit the effect of epitope spread to additional peptides on the myelin proteins. The T cell vaccine provided herein may be individualized for any given patient based on the variability and promiscuity of autoreactive T cell receptors among patients.

### 3. Epitope Screening Analysis (EAA)

A method of identifying an epitope of an autoreactive polypeptide is disclosed. A sample comprising T cells isolated from a host is provided. For example, peripheral blood mononuclear cells (PBMCs) or mononuclear cells from the cerebrospinal fluid (CSFMCs) may be collected from a host. The sample may then be divided into a plurality of portions, each of which may be incubated in the presence of one or more different peptides or a control. The sequences of the peptides collectively comprise the complete sequence of the polypeptide antigen. Finally, a portion of the sample may be identified comprising stimulated autoreactive T cells. The portion of the sample comprising stimulated autoreactive T cells may be identified by reference to a stimulation index (SI).

The EAA may result in growth of both CD4 (MHC II) and CD8 (MHC I). Originally, Th1 CD4 class II cells were thought to be the only pathogenic cells in MS; however, it has become evident that CTL CD8 class I cells are also strongly associated with MS. Some have described that MHC II loci are the predominant genetic link and others have shown that in the background of the MHC II loci there is a strong association with certain MHC I loci for MS and more severe forms of MS. Previous predictive methodologies do not identify the peptides across both MHC II and I. The EAA identifies these peptides without the need to know the MHC II and I relationships. Furthermore, the EAA may use a peptide of sufficient length to tailor the vaccine for the patient. APCs may process a peptide to yield a 10 to 11 amino acid peptide that is presented by the Class II MHC. Peptides may undergo partial proteolysis that yields a sequence that may be bound to Class I MHC, which may be about 9 amino acids in length. As a result, CD4 and CD8 T-cells may grow out by using a suitable peptide if indeed MHC I and MHC II are associated with MS in a particular patient.

### a. Peptides

Peptides comprising a portion of any autoreactive polypeptide may be used in the screening method. The peptides may comprise a portion of MS-associated autoreactive polypeptides such as MBP (NCBI accession number P02686, or a polypeptide substantially identical thereto), MOG (NCBI accession number CAA52617, or a polypeptide substantially identical thereto), PLP (NCBI accession number AAA60350, or a polypeptide substantially identical thereto), or a combination thereof. The sequences of the peptides may collectively comprise the complete sequence of the polypeptide antigen. The different peptides comprise overlapping sequence of 4 to 19 amino. The peptides are 8 to 20 amino acids.

### b. Stimulation Index (SI)

The SI may be calculated by comparing [³H] thymidine incorporation of the sample in the presence of a peptide comprising a portion of an MRTC polypeptide target to that in the presence of a media only control. Briefly, separate aliquots of the sample are plated and incubated in the presence of either a peptide comprising a portion of the polypeptide or a media only control in order to stimulate reactive T cells. The cultures are pulsed with [³H] thymidine during the last 6-18 hours of incubation. The SI is calculated as the quotient of the mean counts per minute (cpm) of the antigen aliquots / mean cpm of the control aliquots. The SI of autoreactive T cells may be increased by at least a factor of 1.3, 1..4, 1.5, 1.6, 1.7, 1.8, 1.9, 2.0, 2.1, 2.2, 2.3, 2.4, 2.5, 2.6, 2.7. 2.8, 2.9, 3.0, 3.1, 3.2, 3.3, 3.4, 3.5, 3.6, 3.7, 3.8, 3.9, or 4.0 compared to a control.

### (1) Predetermined Value

The SI may predetermined value is 1.3, 1.4, 1.5, 1.6, 1.7, 1.8, 1.9, 2.0, 2.1, 2.2, 2.3, 2.4, 2.5, 2.6, 2.7. 2.8, 2.9, 3.0, 3.1, 3.2, 3.3, 3.4, 3.5, 3.6, 3.7, 3.8, 3.9, or 4.0.

The predetermined value may also be calculated using a Variance Evaluation Method as follows:
1. EAAs may be run on a given patient population (minimum of 120 assays from 120 unique subjects)
2. A "negative population" may be created by averaging all SI values < 2.5
3. The Standard Deviation (SD) may be calculated for each peptide
4. The following for each peptide may be then be calculated:
   a. Average + 1SD
   b. Average + 2SD
   c. Average + 3SD
5. The following SIs may be considered above the predetermined value:
   a. SI greater than or equal to the average + 3SD
   b. Any SI greater than or equal to 2.5

The SI may also be calculated by testing each well instead of averaging wells performed in triplicate. In this case, any single well meeting the above criteria may deem the assay above the predetermined value.

The predetermined value may also be evaluated using a Counts per Minute (CPM) Variance Method. This algorithm may set a comprehensive cutoff for all peptides within a given assay. It may be calculated as follows:
1. EAAs may be run and the CPM for all control (media only, no antigen) wells averaged.
2. The SD for CPM of the control wells may then be calculated.
3. The following may then be calculated for the control wells:
   a. Average + 1SD
   b. Average + 2SD
   c. Average + 3SD
4. Any peptide with an average CPM greater than or equal to the average + 3SD of the control wells may be considered above the predetermined value.

Each well may also be evaluated instead of averaging wells performed in triplicate. In this case, any single well meeting the above criteria may deem the assay above the predetermined value.

Any peptide, either analyzed in an individual well or as averaged over wells run in triplicate, with an SI ≥ 2.5, an SI ≥ average +3SD by the Variance Evaluation Method, or an SI ≥ average +3SD by the CPM variance method may be considered above the predetermined value, which may indicate a T cell(s) positive for reactivity to a peptide.

### 4. Method of Making a T Cell Vaccine

A method of preparing a T cell vaccine is disclosed, A sample comprising T cells isolated from a patient may be provided. A peptide comprising an epitope identified by the screening method may then be added. Autoreactive T cells may then be isolated. The T cells may then be attenuated.

### a. Stimulation

Autologous T cells identified as having an S.I. above a predetermined value for a peptide comprising an epitope identified by the screening method may be subjected to recurrent stimulation cycles with the corresponding peptide, optionally and IL-2, in the presence of APCs such as irradiated autologous PBMCs. Irradiation may be at 3500 or 2500-6000 rads. Stimulation cycles may be carried out for 7-14 days. The stimulation cycles may also be carried out for 7-10 days. The T cells may be propagated in stimulation cycles until the total cell number reaches a therapeutic level. The T-cell lines may be cryopreserved at this point.

The T-cells may be activated by non-specific stimulation to induce the upregulation of ergotopes. The resulting activated T cells may then be attenuated. The T cells may be attenuated by any method which makes the T cells replication incompetent yet viable. For example, the T cells may be attenuated by irradiation such as gamma irradiation or by chemical inactivation.

### b. Activation

Autoreactive T cells may be activated during growth cycles of stimulation prior to attenuation. Activation of T cells during growth cycles prior to attenuation may induce a general up regulation of ergotopes expressed on the surface of activated but not resting T cells (Irun R. Cohen, Francisco J. Quintana, and Avishai Mimran. Tregs in T cell vaccination: exploring the regulation of regulation. JCI Volume 114 (9) 1227-1232, 2004). Thus, when autoreactive T cells are grown as activated T cells prior to attenuation both anti-ergotypic and anti-idiotypic T cell responses may be expected following vaccination. (Cohen et al., JCI Volume 114 (9): 1227-1232, 2004). Autoreactive T cells may be activated by exposure to mitogens (such as phytohemaggluti.nin (PHA)) or interleukin-2 in the presence of PHA or through ligation of the TCR/CD3 complex (Kobayashi et al., J. Exp. Med. 170:827). One of the primary mechanisms through which T cell activation may confer responsiveness to IL-2 is through the up-regulation of the receptor subunits (ergotopes) for these cytokines (Chua et al., J. Immunol. 153:128, 1994; Desai et al., J. Immunol. 148:3125, 1992; Presky et al., Proc. Natl. Acad. Sci. USA 93:14002, 1997; and Wu et al., Eur. J. Immunol. 27:147, 1997).

### c. APC

The APCs may be white blood cells. Representative examples of APCs include monocytes, dendritic cells and B cells.

### d. T cells

The T cell vaccine may comprise T cells positive for cell markers CD3, CD4, CD8, CD25, TCR αβ, TCR γδ, HSP60 (heat-shock protein 60) or a combination thereof. The T cell vaccine may also comprise T cell membranes or fragments thereof.

### e. Attenuation

The T cell vaccine may be attenuated. Attenuated may be by any method that makes the T cells replication incompetent yet viable. For example, the T cells may be attenuated by irradiation such as gamma irradiation or by chemical inactivation. The gamma irradiation may be 10,000 or 7,000-12,000 rads.

### 5. T Cell Vaccines

A T cell vaccine is also disclosed. The vaccine may be produced as described above. The vaccine may comprise T cells that are specific to an antigenic polypeptide characterized in that epitopes (optionally, all epitopes) of the antigenic polypeptide capable of producing a stimulation index above a predetermined value are recognized by autoreactive T cells present in the vaccine. The vaccine may comprise 60-90 million, 30-45 million, or 6-9 million T cells. The vaccine may also comprise less than 50% T cells that recognize the epitopes of the antigenic polypeptide capable of producing a stimulation index of less than the predetermined value. The vaccine may also comprise less than 45%, 40%, 35%, 30%, 25%, 20%, 15%, 10%, 5%, 4%, 3%, 2% or 1% T cells that recognize the epitopes of the antigenic polypeptide capable of producing a stimulation index of less than predetermined value.

The T cell vaccine may comprise T cells that are specific to multiple antigenic polypeptides. For example, a T cell vaccine to be administered to a patient with MS may contain T cells specific to MBP, PLP and MOG.

### 6. Method of Detecting Epitope Shift

A method of detecting an epitope shift in a T cell mediated disease is disclosed. The method comprises identifying the epitopes of an autoreactive antigen using the screening method and comparing those epitopes to a control. If the epitopes are different, an epitope shift has been detected. Detecting an epitope shift may be used to diagnose an epitope shift or to monitor epitope shift.

By providing peptides comprising a portion of the sequence of a polypeptide antigen, the invention provides a method of identifying cryptic epitopes that are masked in the full length protein

The present invention has multiple aspects, illustrated by the following non-limiting examples.

### Example 1

### Immunodominant-Based Vaccines

In previous clinical trials, vaccine cell lines were produced by stimulating T cell cultures with two immunodominant peptides from MBP. In recent clinical trials, vaccine cell lines were produced by stimulating T-cells with a total of six immunodominant peptides (2 from MBP, 2 from PLP and 2 from MOG). For some patients, use of this limited set of peptides resulted in poorly growing cultures and several weeks to produce the vaccine. T cells were not being optimally stimulated because the peptides were not optimally matched to the HLA phenotype for every patient. In addition, the majority of the epitopes involved are not covered by using only suspected immunodominant epitopes and hence a truly individualized vaccine is not achieved. Epitopes that are not covered may be stimulating clonal expansion and pathogenesis *in vivo* and may be unchecked.

### Example 2

### Myelin Epitopes

In order to determine whether the choice of proper stimulatory peptides could be improved, additional peptide epitopes within MBP, PLP and MOG were prepared and tested in multiple sclerosis patients. To perform the analysis, a total of 163 different overlapping peptides (the synthesis of each peptide of 16 amino acids (16-mer) is offset by 4 amino acids with an overlap of 12 amino acids of the previous sequence) that covered the full length of MBP, PLP and MOG were synthesized. A total of 44 MBP, 67 PLP and 52 MOG peptide sequences were synthesized. The list of sequences, their identifiers and amino acid number and how they were combined for use in the EAA (Mix ID) is shown in Tables 1-3. The six immunodominant sequences used in Example 1 are bolded.

**Table 1 MBP Sequences**

| Peptide Mix ID | Peptide Seq ID | Amino Acids | | | Sequence | SEQ ID NO |
|---|---|---|---|---|---|---|
| | MBP | 1 | to | 16 | MASQKRPSQRHGSKYL | 1 |
| MBPm1 | MBP 2 | 5 | to | 20 | KRPSQRHGSKYLATAS | 2 |
| | MBP 3 | 9 | to | 24 | QRHGSKYLATASTMDH | 3 |
| MBPm2 | MBP 4 | 13 | to | 28 | SKYLATASTMDHARHG | 4 |
| | MBP 5 | 17 | to | 32 | ATASTMDHARHGFLPR | 5 |
| MBPm3 | MBP 6 | 21 | to | 36 | TMDHARHGFLPRHRDT | 6 |
| | MBP 7 | 25 | to | 40 | ARHGFLPRHRDTGILD | 7 |
| MBPm4 | MBP 8 | 29 | to | 44 | FLPRHRDTGBLDSIGR | 8 |
| | MBP 9 | 33 | to | 48 | HRDTGILDSIGRFFGG | 9 |
| MBPm5 | MBP 10 | 37 | to | 52 | GILDSIGRFFGGDRGA | 10 |
| | MBP 11 | 41 | to | 56 | SIGRFFGGDRGAPKRG | 11 |
| MBPm6 | MBP 12 | 45 | to | 60 | FFGGDRGAPKRGSGKV | 12 |
| | MBP 13 | 49 | to | 64 | DRGAPKRGSGKVPWLK | 13 |
| MBPm7 | MBP 14 | 53 | to | 68 | PKRGSGKVPWLKPGRS | 14 |
| | MBP 15 | 57 | to | 72 | SGKVPWLKPGRSPLPS | 15 |
| MBPm8 | MBP 16 | 61 | to | 76 | PWLKPGRSPLPSHARS | 16 |
| | MBP 17 | 65 | to | 80 | PGRSPLPSHARSQPGL | 17 |
| MBPm9 | MBP 18 | 69 | to | 84 | PLPSHARSQPGLCNMY | 18 |
| | MBP 19 | 73 | to | 88 | HARSQPGLCNMYKDSH | 19 |
| MBPm10 | MBP 20 | 77 | to | 92 | QPGLCNMYKDSHHPAR | 20 |
| | MBP 21 | 81 | to | 96 | CNMYKDSHHPARTAHY | 21 |
| MBPm11 | MBP 22 | 85 | to | 100 | KDSHHPARTAHYGSLP | 22 |
| | MBP 23 | 89 | to | 104 | HPARTAHYGSLPQKSH | 23 |
| MBPm12 | MBP 24 | 93 | to | 108 | TAHYGSLPQKSHGRTQ | 24 |
| | MBP 25 | 97 | to | 112 | GSLPQKSHGRTQD**ENP** | 25 |
| MBPm13 | MBP 26 | 101 | to | 116 | QKSHGRTQD**ENPVVHF** | 26 |
| | MBP 27 | 105 | to | 120 | GRTQD**ENPVVHFFKNI** | 27 |
| MBPm14 | MBP 28 | 109 | to | 124 | D**ENPVVHFFKNIVTPR** | 28 |
| | MBP 29 | 113 | to | 128 | **VVHFFKNIVTPRTP**PP | 29 |
| MBPm15 | MBP 30 | 117 | to | 132 | **FKNIVTPRTP**PPSQGK | 30 |
| | MBP 31 | 121 | to | 136 | **VTPRTP**PPSQGGAEG | 31 |
| MBPm16 | MBP 32 | 125 | to | 140 | **TP**PPSQGGAEGQRPG | 32 |
| | MBP 33 | 129 | to | 144 | SQGGAEGQRPGFGYG | 33 |
| MBPm17 | MBP 34 | 133 | to | 148 | AEGQRPGFGYGGRAS | 34 |
| | MBP 35 | 137 | to | 152 | RPGFGYGGRASDYKS | 35 |
| MBPml8 | MBP 36 | 141 | to | 156 | GYGGRASDYKS AHKG | 36 |
| | MBP 37 | 145 | to | 160 | RASDYKSAHKGFKGV | 37 |
| MBPml9 | MBP 38 | 149 | to | 164 | YKSAHKGFKGVDAQG | 38 |
| | MBP 39 | 153 | to | 168 | HKGFKGVDAQGTL**SK** | 39 |
| MBPm20 | MBP 40 | 157 | to | 172 | KG VDAQGTLS**KIFKL** | 40 |
| | MBP 41 | 161 | to | 176 | AQGTL**SKIFKLGGRD** | 41 |
| MBPm21 | MBP 42 | 165 | to | 180 | L**SKIFKLGGRDSRSG** | 42 |
| | MBP 43 | 169 | to | 184 | **FKLGGRDSRSGSPMA** | 43 |
| MBPm22 | MBP 44 | 173 | to | 185 | **GRDSRSGSPMARR** | 44 |

**Table 2 PLP Sequences**

| Peptide Mix ID | Peptide Seq ID | Amino Acids | | | Sequence | SEQ ID NO |
|---|---|---|---|---|---|---|
| | PLP 1 | 1 | to | 16 | MGLLECCARCLVGAPF | 45 |
| PLPm1 | PLP 2 | 5 | to | 20 | ECCARCLVGAPFASLV | 46 |
| | PLP 3 | 9 | to | 24 | RCLVGAPFASLVATGL | 47 |
| PLPm2 | PLP 4 | 13 | to | 28 | GAPFASLVATGLCFFG | 48 |
| | PLP 5 | 17 | to | 32 | ASLVATGLCFFGVA**LF** | 49 |
| PLPm3 | PLP 6 | 21 | to | 36 | ATGLCFFGVA**LFCGCG** | 50 |
| | PLP 7 | 25 | to | 40 | CFFGVA**LFCGCGHEAL** | 51 |
| PLPm4 | PLP 8 | 29 | to | 44 | VA**LFCGCGHEALTGTE** | 52 |
| | PLP 9 | 33 | to | 48 | **CGCGHEALTGTEKLIE** | 53 |
| PLPm5 | PLP 10 | 37 | to | 52 | **HEALTGTEKLIETYFS** | 54 |
| | PLP 11 | 41 | to | 56 | **TGTEKLIETY**FSKNYQ | 55 |
| PLPm6 | PLP 12 | 45 | to | 60 | **KLIETY**FSKNYQDYEY | 56 |
| | PLP 13 | 49 | to | 64 | **TY**FSKNYQDYEYLINV | 57 |
| PLPm7 | PLP 14 | 53 | to | 68 | KNYQDYEYLINVIHAF | 58 |
| | PLP 15 | 57 | to | 72 | DYEYLIN VIHAFQY VI | 59 |
| PLPm8 | PLP 16 | 61 | to | 76 | LINVIHAFQYVIYGTA | 60 |
| | PLP 17 | 65 | to | 80 | IHAFQYVIYGTASFFF | 61 |
| PLPm9 | PLP 18 | 69 | to | 84 | QYVIYGTASFFFLYGA | 62 |
| | PLP 19 | 73 | to | 88 | YGTASFFFLYGALLLA | 63 |
| PLPm 10 | PLP 20 | 77 | to | 92 | SFFFLYGALLLAEGFY | 64 |
| | PLP 21 | 81 | to | 96 | LYGALLLAEGFYTTGA | 65 |
| PLPm11 | PLP 22 | 85 | to | 100 | LLLAEGFYTTGAVRQI | 66 |
| | PLP 23 | 89 | to | 104 | EGFYTTGAVRQIFGDY | 67 |
| PLPm12 | PLP 24 | 93 | to | 108 | TTGAVRQIFGDYKTTI | 68 |
| | PLP 25 | 97 | to | 112 | VRQIFGDYKTTICGKG | 69 |
| PLPm13 | PLP 26 | 101 | to | 116 | FGDYKTTICGKGLSAT | 70 |
| | PLP 27 | 105 | to | 120 | KTTICGKGLSATVTGG | 71 |
| PLPm14 | PLP 28 | 109 | to | 124 | CGKGLSATVTGGQKGR | 72 |
| | PLP 29 | 113 | to | 128 | LSATVTGGQKGRGSRG | 73 |
| PLPm15 | PLP 30 | 117 | to | 132 | VTGGQKGRGSRGQHQA | 74 |
| | PLP 31 | 121 | to | 136 | QKGRGSRGQHQAHSLE | 75 |
| PLPm16 | PLP 32 | 125 | to | 140 | GSRGQHQAHSLERVCH | 76 |
| | PLP 33 | 129 | to | 144 | QHQAHSLERVCHCLGK | 77 |
| PLPm17 | PLP 34 | 133 | to | 148 | HSLERVCHCLGKWLGH | 78 |
| | PLP 35 | 137 | to | 152 | RVCHCLGKWLGHPDKF | 79 |
| PLPm18 | PLP 36 | 141 | to | 156 | CLGKWLGHPDKFVGIT | 80 |
| | PLP 37 | 145 | to | 160 | WLGHPDKFVGITYALT | 81 |
| PLPm19 | PLP 38 | 149 | to | 164 | PDKFVGITYALTVVWL | 82 |
| | PLP 39 | 153 | to | 168 | VGITYALTVVWLLVFA | 83 |
| PLPm20 | PLP 40 | 157 | to | 172 | YALTVVWLLVFACSAV | 84 |
| | PLP 41 | 161 | to | 176 | VVWLLVFACSAVPVYI | 85 |
| PLPm21 | PLP 42 | 165 | to | 180 | LVFACSAVPVYIYFNT | 86 |
| | PLP 43 | 169 | to | 184 | CSAVPVYIYFNT**WTTC** | 87 |
| PLPm22 | PLP 44 | 173 | to | 188 | PVYIYFNT**WTTCQSIA** | 88 |
| | PLP 45 | 177 | to | 192 | YFNT**WTTCQSIAFPSK** | 89 |
| PLPm23 | PLP 46 | 181 | to | 196 | **WTTCQSIAFPSKTSAS** | 90 |
| | PLP 47 | 185 | to | 200 | **QSIAFPSKTSASIGSL** | 91 |
| PLPm24 | PLP 48 | 189 | to | 204 | **FPSKTSASIGSL**CADA | 92 |
| | PLP 49 | 193 | to | 208 | **TSASIGSL**CADARMYG | 93 |
| PLPm25 | PLP 50 | 197 | to | 212 | **IGSL**CADARMYGVLPW | 94 |
| | PLP 51 | 201 | to | 216 | CADARMYGVLPWNAFP | 95 |
| PLPm26 | PLP 52 | 205 | to | 220 | RMYGVLPWNAFPGKVC | 96 |
| | PLP 53 | 209 | to | 224 | VLPWNAFPGKVCGSNL | 97 |
| PLPm27 | PLP 54 | 213 | to | 228 | NAFPGKVCGSNLLSIC | 98 |
| | PLP 55 | 217 | to | 232 | GKVCGSNLLSICKTAE | 99 |
| PLPm28 | PLP 56 | 221 | to | 236 | GSNLLSICKTAEFQMT | 100 |
| | PLP 57 | 225 | to | 240 | LSICKTAEFQMTFHLF | 101 |
| PLPm29 | PLP 58 | 229 | to | 244 | KTAEFQMTFHLFIAAF | 102 |
| | PLP 59 | 233 | to | 248 | FQMTFHLFIAAFVGAA | 103 |
| PLPm30 | PLP 60 | 237 | to | 252 | FHLFIAAFVGAAATLV | 104 |
| | PLP 61 | 241 | to | 256 | IAAFVGAAATLVSLLT | 105 |
| PLPm31 | PLP 62 | 245 | to | 260 | VGAAATLVSLLTFMIA | 106 |
| | PLP 63 | 249 | to | 264 | ATLVSLLTFMIAATYN | 107 |
| PLPm32 | PLP 64 | 253 | to | 268 | SLLTFMIAATYNFAVL | 108 |
| | PLP 65 | 257 | to | 272 | FMIAATYNFAVLKLMG | 109 |
| PLPm33 | PLP 66 | 261 | to | 276 | ATYNFAVLKLMGRGTK | 110 |
| PLP67 | PLP 67 | 265 | to | 277 | FAVLKLMGRGTKF | 111 |

**Table 3 MOG Sequences**

| Peptide Mix ID | Peptide Seq ID | Amino Acids | | | Sequence | SEQ ID NO |
|---|---|---|---|---|---|---|
| | MOG 1 | 1 | to | 16 | **GQFRVIGPRHPIRALV** | 112 |
| MOGm1 | MOG 2 | 5 | to | 20 | **VIGPRHPIRALVGDEV** | 113 |
| | MOG 3 | 9 | to | 24 | **RHPIRALVGDEVELPC** | 114 |
| MOGm2 | MOG 4 | 13 | to | 28 | **RALVGDEVELPCRISP** | 115 |
| | MOG 5 | 17 | to | 32 | **GDEVELPCRISPGKNA** | 116 |
| MOGm3 | MOG 6 | 21 | to | 36 | **ELPCRISPGKNATGME** | 117 |
| | MOG 7 | 25 | to | 40 | **RISPGKNATGMEVGWY** | 118 |
| MOGm4 | MOG 8 | 29 | to | 44 | **GKNATBMEVGW**YRPPF | 119 |
| | MOG 9 | 33 | to | 48 | **TGMEVGW**YRPPFSRVV | 120 |
| MOGm5 | MOG 10 | 37 | to | 52 | **VGW**YRPPFSRVVHLYR | 121 |
| | MOG 11 | 41 | to | 56 | RPPFSRVVHLYRNGKD | 122 |
| MOGm6 | MOG 12 | 45 | to | 60 | SRVVHLYRNGKDQDGD | 123 |
| | MOG 13 | 49 | to | 64 | HLYRNGKDQDGDQAPE | 124 |
| MOGm7 | MOG 14 | 53 | to | 68 | NGKDQDGDQAPEYRGR | 125 |
| | MOG 15 | 57 | to | 72 | QDGDQAPEYRGRTELL | 126 |
| MOGm8 | MOG 16 | 61 | to | 76 | QAPEYRGRTELLKDAI | 127 |
| | MOG 17 | 65 | to | 80 | YRGRTELLKDAIGEGK | 128 |
| MOGm9 | MOG 18 | 69 | to | 84 | TFLLKDAIGEGKVTLR | 129 |
| | MOG 19 | 73 | to | 88 | KDAIGEGKVTLRIRNV | 130 |
| MOGm10 | MOG 20 | 77 | to | 92 | GEGKVTLRIRNVRFSD | 131 |
| | MOG 21 | 81 | to | 96 | VTLRIRNVRFSDEGGF | 132 |
| MOGm11 | MOG 22 | 85 | to | 100 | IRNVRFSDEGGFRCFF | 133 |
| | MOG 23 | 89 | to | 104 | RFSDEGGFTCFFRDHS | 134 |
| MOGm12 | MOG 24 | 93 | to | 108 | EGGFTCFFRDHSYQEE | 135 |
| | MOG 25 | 97 | to | 112 | TCFFRDHSYQEEAAME | 136 |
| MOGm13 | MOG 26 | 101 | to | 116 | RDHSYQEEAAMELKVE | 137 |
| | MOG 27 | 105 | to | 120 | YQEEAAMELKVEDPFY | 138 |
| MOGm14 | MOG 28 | 109 | to | 124 | AAMELKVEDPFYWVSP | 139 |
| | MOG 29 | 113 | to | 128 | LKVEDPFYWVSPGVLV | 140 |
| MOGm15 | MOG 30 | 117 | to | 132 | DPFYWVSPGVLVLLAV | 141 |
| | MOG 31 | 121 | to | 136 | WVSPGVLVLLAVLPVL | 142 |
| MOGm16 | MOG 32 | 125 | to | 140 | GVLVLLAVLPVLLLQI | 143 |
| | MOG 33 | 129 | to | 144 | LLAVLPVLLLQITVGL | 144 |
| MOGm17 | MOG 34 | 133 | to | 148 | LPVLLLQITVGLVFLC | 145 |
| | MOG 35 | 137 | to | 152 | LLQITVGLVFLCLQYR | 146 |
| MOGm18 | MOG 36 | 141 | to | 156 | TVGLVFLCLQYRLRGK | 147 |
| | MOG 37 | 145 | to | 160 | VFLCLQYRLRGKLRAE | 148 |
| MOGm19 | MOG 38 | 149 | to | 164 | LQYRLRGKLRAEIENL | 149 |
| | MOG 39 | 153 | to | 168 | LRGKLRAEIENLHRTF | 150 |
| MOGm20 | MOG 40 | 157 | to | 172 | LRAEIENLHRTFDPHF | 151 |
| | MOG 41 | 161 | to | 176 | IENLHRTFDPHFLRVP | 152 |
| MOGm21 | MOG 42 | 165 | to | 180 | HRTFDPHFLRVPCWKI | 153 |
| | MOG 43 | 169 | to | 184 | DPHFLRVPCWKITLFV | 154 |
| MOGm22 | MOG 44 | 173 | to | 188 | LRVPCWKITLFVIVPV | 155 |
| | MOG 45 | 177 | to | 192 | CWKITLFVIVPVLGPL | 156 |
| MOGm23 | MOG 46 | 181 | to | 196 | TLFVIVPVLGPLVALI | 157 |
| | MOG 47 | 185 | to | 200 | IVPVLGPLVALIICYN | 158 |
| MOGm24 | MOG 48 | 189 | to | 204 | LGPLVALIICYNWLHR | 159 |
| | MOG 49 | 193 | to | 208 | VALIICYNWLHRRLAG | 160 |
| MOGm25 | MOG 50 | 197 | to | 212 | ICYNWLHRRLAGQFLE | 161 |
| | MOG 51 | 201 | to | 216 | WLHRRLAGQFLEELRN | 162 |
| MOGm26 | MOG 52 | 205 | to | 218 | RLAGQFLEELRNPF | 163 |

### Example 3

### Generating a Myelin Antigen Repertoire

Overlapping peptides spanning the MBP protein that are each 16aa in length and overlap by 12aa were generated. All MBP peptides could be manufactured, however, the repertoire of MBP peptides excluded eight peptides. The resulting 36 peptides cover 95.7% of the protein. Only amino acids 1-8 were not covered. The list of MBP peptides is in Table 4 with the peptides not used highlighted in light grey.

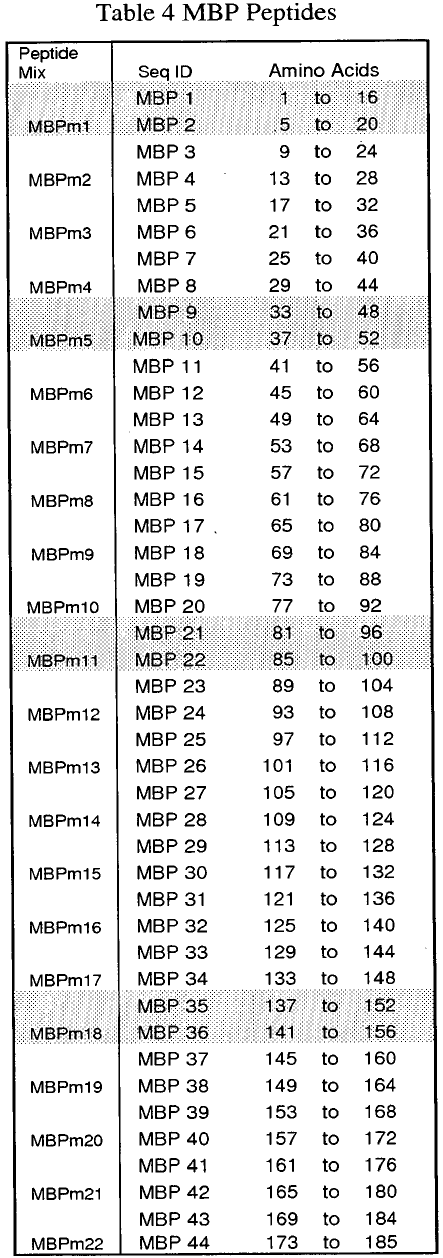

Overlapping peptides spanning the PLP protein that are each 16aa in length and overlap by 12aa were also generated. Not all PLP peptides could be manufactured, including the sequences from amino acids 61-72 and 245-248. In addition, the PLP peptide repertoire excluded 20 peptides. The resulting 35 peptides cover 83.0% of the protein. The only regions not covered were amino acids 21-24, 61-80, 117-128, 165-168 and 237-248. The list of PLP peptides is shown in Table 5 , with the peptides not used highlighted in light grey and those not able to be manufactured in stippled shading.

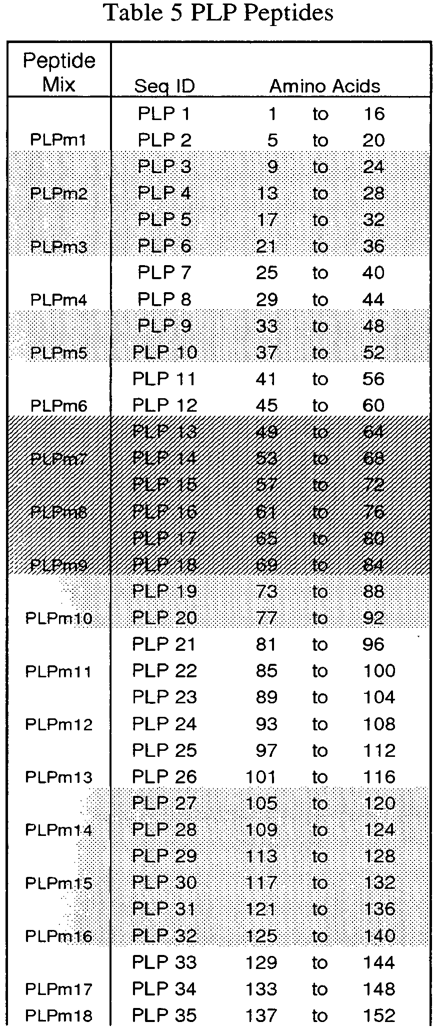

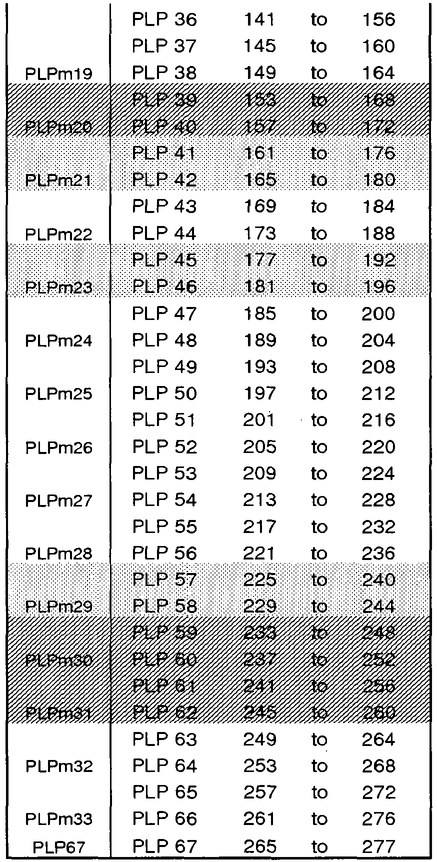

Overlapping peptides spanning the MOG protein that are each 16aa in length and overlap by 12aa were also generated. Not all MOG peptides could be manufactured, including the sequence from amino acids 141-144. In addition, the MOG peptide repertoire excluded eight peptides. The resulting 40 peptides cover 93.6% of the protein. The only regions not covered were amino acids 69-80 and 141-144. The list of MOG peptides is shown in Table 6 , with the peptides not used highlighted in light grey and those not able to be manufactured in stippled shading.

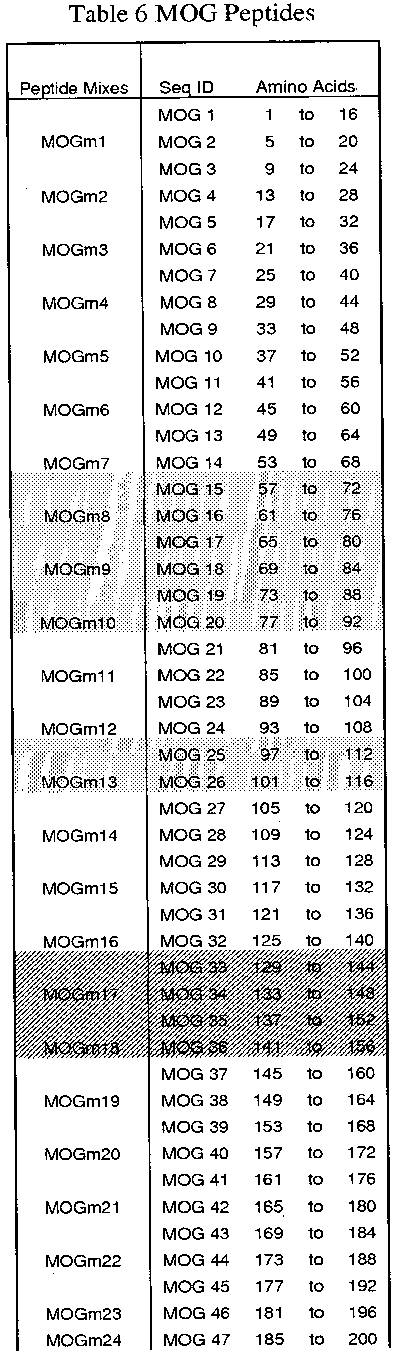

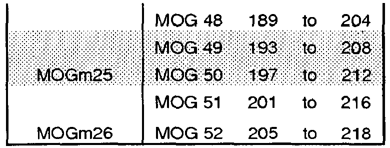

Peptides were synthesized for the entire length of MBP, PLP, and MOG with purities of >95% in most cases. All peptides that could be synthesized were evaluated in subsequent experiments. At total of 16 peptides could not be synthesized by solid-phase peptide synthesis (SPPS). These 16 peptides covered a unique span of 18 amino acids over the 3 proteins (2.6% of the total protein content). All peptides not able to be manufactured were hydrophic in nature.

### Example 4

### Epitope Analysis Assay

The peptides in Example 2 were tested in an *in vitro* PBMC stimulation assay to identify myelin reactive T cells in a patient's blood. Peripheral blood mononuclear cells (PBMCs) were separated from whole blood, washed, counted and plated at 250,000 cells per well in a total of four 96-well plates. Myelin peptide mixes of two overlapping 16-mer peptides were added to triplicate wells of PBMCs with triplicate media only control wells included on each plate and then incubated. After two days of incubation, 20 U/ml of interleukin-2 (IL-2) was added. On the fifth day, the plates were labeled with a radioisotope (tritiated thymidine) and harvested 6 hours later. In this assay, the cells that incorporate tritiated thymidine are representative of T cells being activated and induced to proliferate by the T cell receptor-peptide-MHC complexes. T cells incorporating comparatively more tritiated thymidine than control and experimental cells are more highly activated T cells and are proliferating more rapidly.

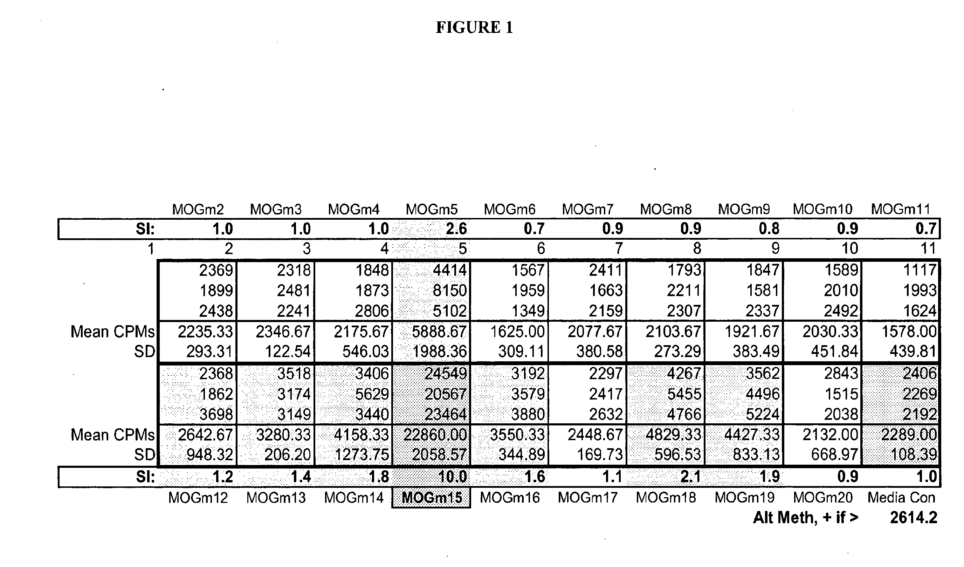

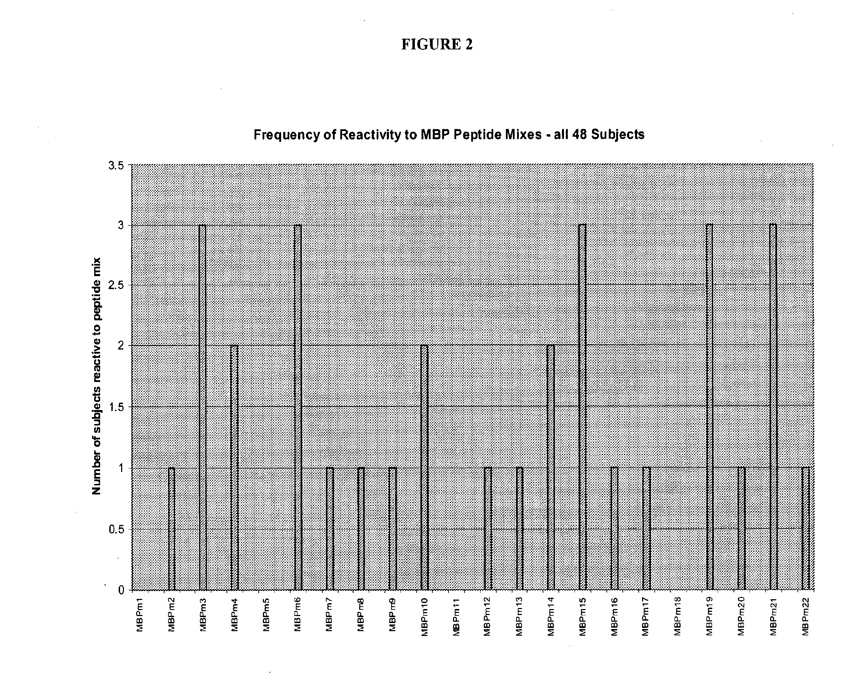

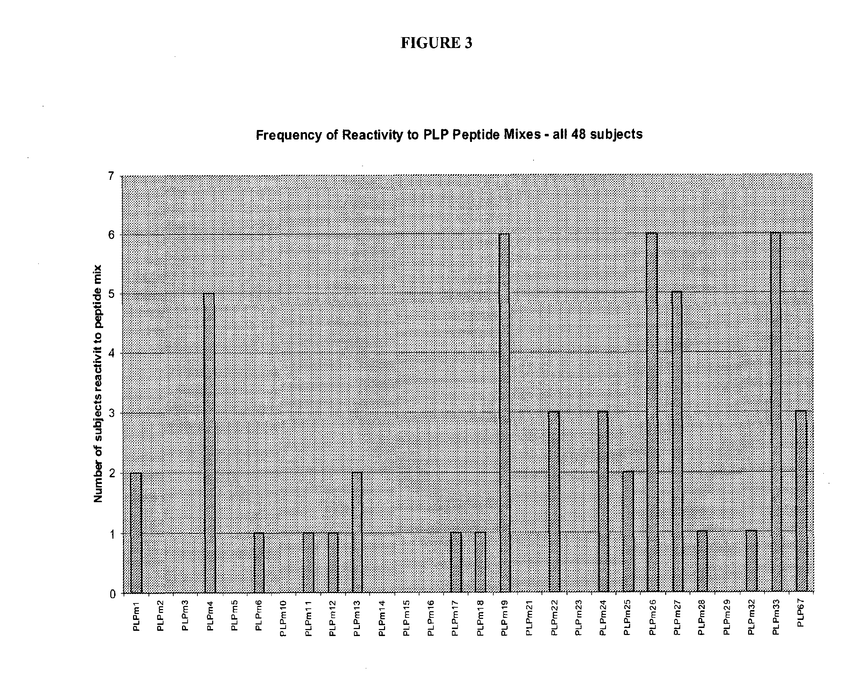

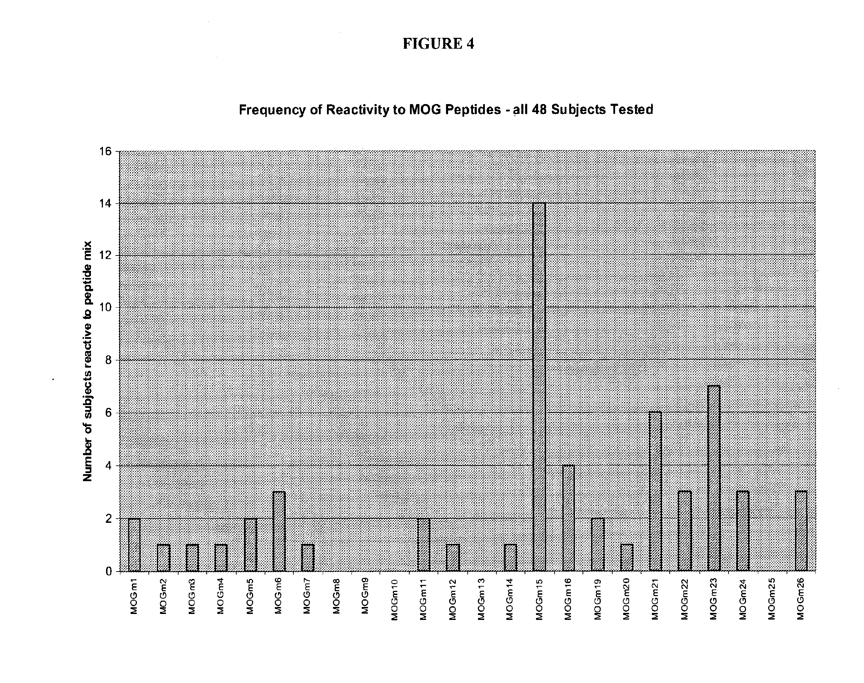

Stimulation Indices (SI) were determined for each peptide mix by dividing the mean radiolabel counts per minute (CPM) of the peptide stimulated wells by the mean CPM of the media only control wells averaged across all four plates. An SI of at least 3 was considered positive. Figure 1 is an example of EAA from one MS patient. Seven of the peptide mixtures were slightly reactive, with the MOGm15 stimulated wells being very reactive. Figures 2-4 show the frequency of reactivity to the peptide mixes in 48 patients.

### Example 5

### HLA Analysis

The active peptides identified by EAA, as described in Example 2, were compared to the HLA phenotype of the patient. An algorithm for predicting class II binding regions available at http://www.imtech.res.in/raghava/propred/ (Singh, H. and G.P.S. Raghava, ProPred: prediction of HLA-DR binding sites) was used to predict binding regions of MOG based on the patient's HLA-DR haplotype of DRB1_0801, DRB1_1501 and DRB5_0101.

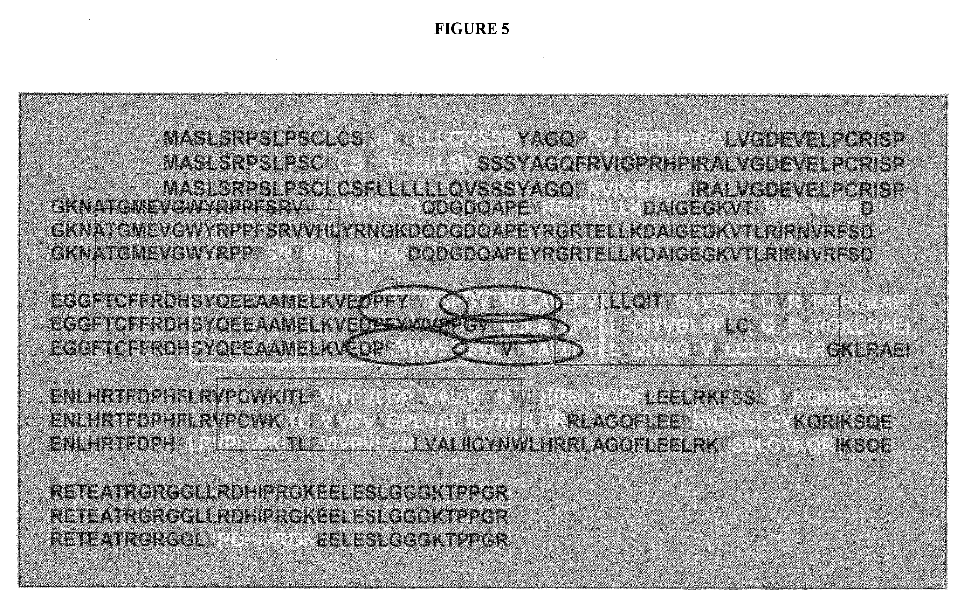

Figure 5 shows the ProPred binding predictions within MOG protein for the 3 HLA alleles of the patient. The red amino acid residues represent predicted anchor residues for binding within the HLA groove, while the yellow residues represent the other residues that would fit within the groove. As a result, these residues would be predicted to be candidate stimulatory epitopes for T cells from this patient.

The bright yellow box surrounds the sequences included in peptide mix MOGm15 that gave the SI of 10. There are sequences within these peptides that are predicted to bind to all three of the HLA alleles. For two of the alleles there are two predicted binding epitopes and even a part of a third within these sequences. Although the predicted binding of these sequences correlated to a certain extent with the results obtained in the EA assay, there are stimulatory peptides that are not predicted.

These results show that EAA provides superior predictive results for identifying patient specific stimulatory peptides. The superiority of EAA is enhanced in view of HLA expression variants. Comparative studies between serology and molecular typing for HLA-A and B loci have discovered alleles detected by DNA typing reagents but not detected by serologic reagents. These HLA expression variants are not expressed or expressed in very low amounts on the cell surface. EAA is superior to software screens because there is no need to identify the HLA variants.

### Example 6

### Production of Vaccine

Patient-specific peptides identified by EAA were tested to determine whether they could be used to produce and expand myelin-reactive T-cells for use in a vaccine. A 500 ml bag of blood was obtained from the patient to set up vaccine production cultures. Bulk cultures were set up in AIM V medium along with the optimized peptides. After 48 hours of incubation, rIL-2 was added at 20 U/ml. Seven days later, the cultures were re-stimulated with PBMCs and peptide. The media was changed to 2% AB serum with 100 U/ml rIL2 in X Vivo 15 medium. The cultures were fed and split every 1-4 days as necessary. After an additional seven days, the cultures were once again stimulated with APCs and peptides. The cultures were continued to be fed and split every 1-4 days as necessary. After an additional 7-14 days, the cultures had expanded to at least 100 x 10⁶ cells. The dells were divided into aliquots of 10 x 10⁶ cells and frozen.

### Example 7

### TCR V_{B} Analysis

The vaccine produced in Example 6 was tested to determine whether the selectively expanded T cells had a particular subset of T cells. Enrichment of T cell subsets was evaluated by analyzing the T cell receptor variable beta chain usage (Vβ). The 24 different known beta chain variable (Vβ) region families were evaluated using specific fluorescent-labeled monoclonal antibodies and flow cytometry. If a particular subset of T cells is selectively expanded by stimulation with a peptide, an increase would be detected in the percentage of cells expressing one of the V beta families as that subset expands.

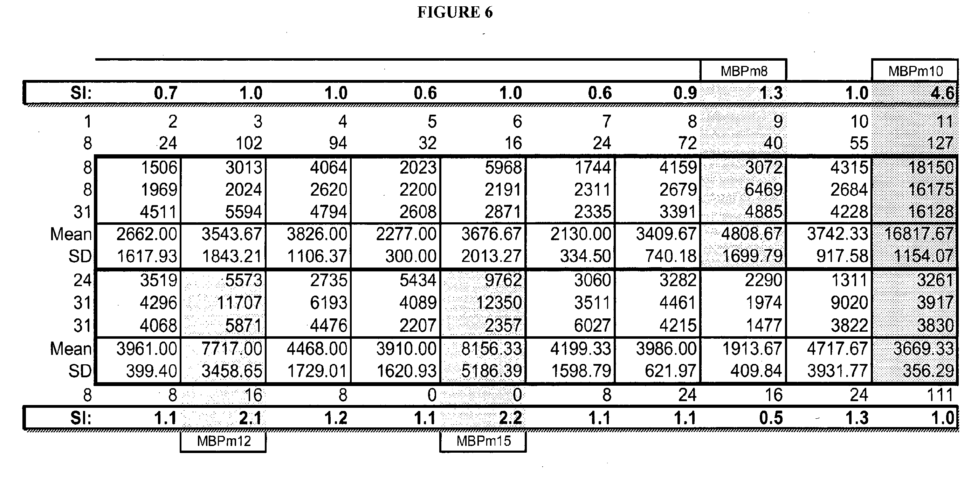

A T cell receptor (TCR) Vbeta analysis was performed after approximately 18 days into the production of a vaccine produced as described in Example 6. A T cell line was produced by stimulating the patient's PBMCs with peptide mix MBPm10, which had produced an SI of 4.6 in the original EAA for this patient. Figure 6 shows that the peptide mix MBPm10 produced an SI of 4.6. This mix was used to stimulate PBMCs from this patient in culture.

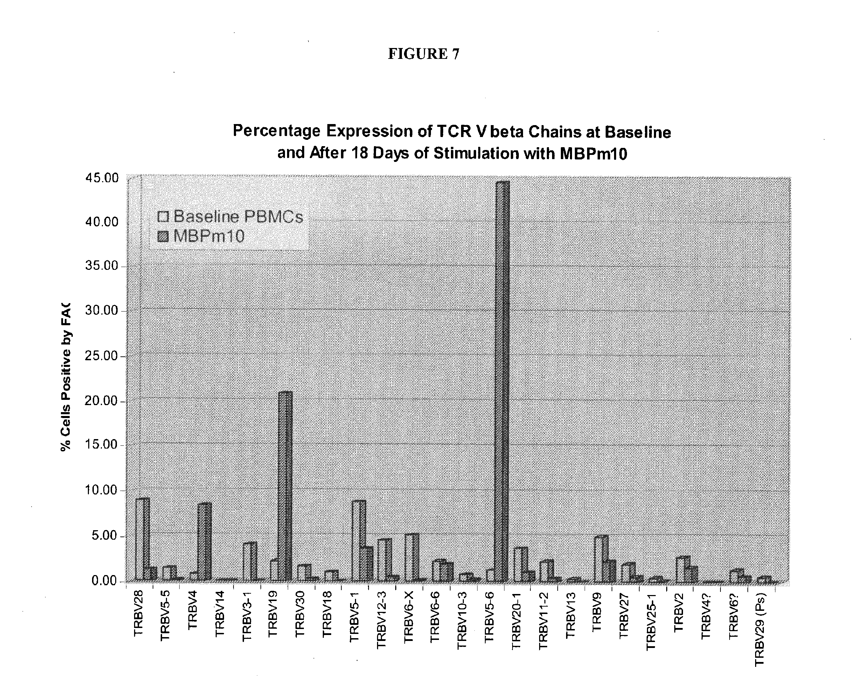

Figure 7 shows the Vbeta analysis performed on the cells at baseline, on PBMCs, and on Day 18 of the T cell culture with MBPm10 peptides for this same patient. At baseline there is the typical relatively even distribution of TCR Vbeta chain usage in the PBMCs. However, after 18 days of stimulation in culture with MBPm10, V beta 5-6 positive T cells now make up 45% of the cells in culture, indicating that stimulation with the MBPm10 peptide mix has been able to focus, or selectively expand, a subset of T cells within the PBMCs of this patient.

### Example 8

### Growth Analysis

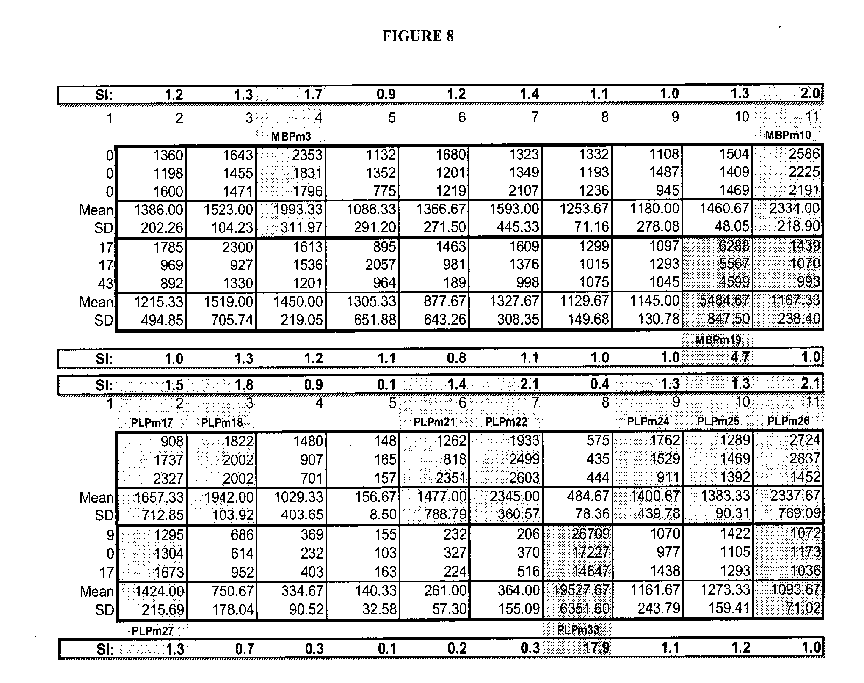

The ability of stimulation with the EAA-selected peptides to more rapidly expand the stimulated the T cells were tested. The cell growth curves were analyzed for two different stimulatory peptide mixes. The EAA CPM data shown in Figure 8 show a strongly reactive mix in MBPm19 for a patient with an SI of 4.7. A second plate from the same assay shows another reactive mix in PLPm33 with a high SI of 17.9. These two peptides mixes were then used to stimulate PBMCs from this patient to produce T cell lines.

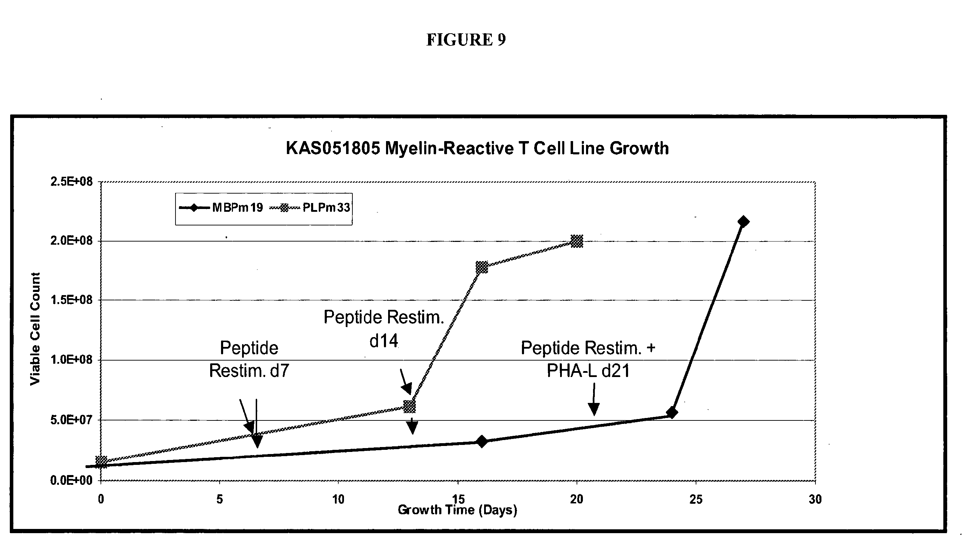

The growth analysis of the EAA-identified peptides is shown in Figure 9. The PLPm33 stimulated cells expanded from 15 million PBMCs to 200 million T cells in 20 days with 3 stimulations with the peptide mix. The MBPm19 stimulated cells were slower to being rapidly expanding, but they also expanded from 15 million PBMCs to 217 million T cells following an additional restimulation with peptide at day 21. They were harvested on day 27 from the day of culture initiation. This process represents a 6-fold shorter process than previous production methods using only immunodominant peptides from MBP, PLP and MOG.

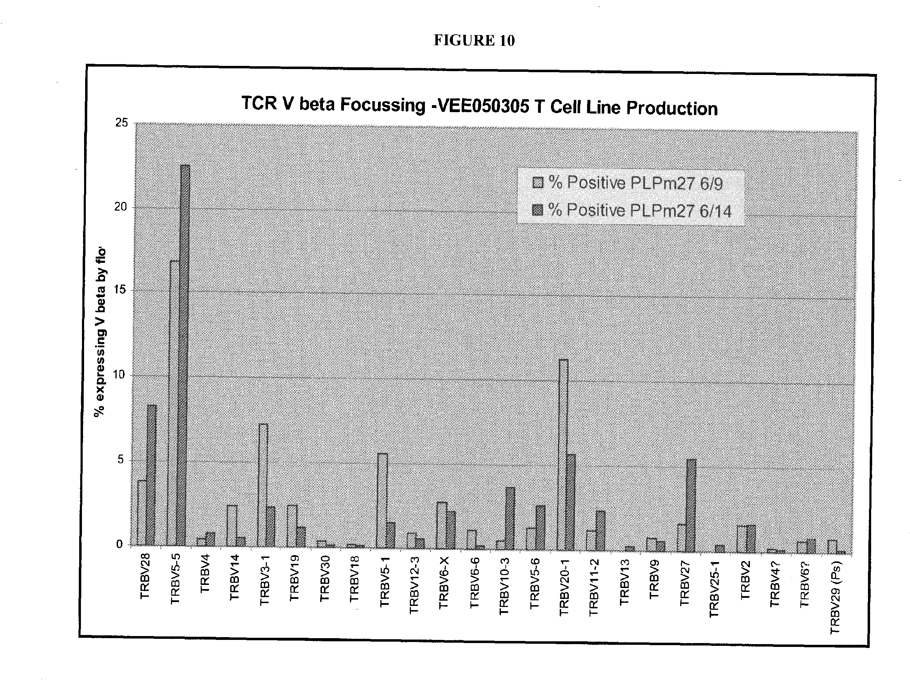

For another MS patient, a total of 8.25 x 10⁶ PBMCs were originally seeded for each stimulation condition. By 21 days in culture, following a total of 3 stimulations with peptide (no PHA used), one cell line had expanded to 142 x 10⁶ cells, while the other cell line had expanded to 95 x 10⁶ T cells. Figure 10 shows the TCR V beta focusing that occurred as the culture progressed from Day 9 to Day 16 in culture, while being stimulated by the peptide mix PLPm27. The T cell subset using V beta chain 5-5 is expanding at the expense of most of the other V beta T cell subsets.

### Example 9

### Monitoring MRTC

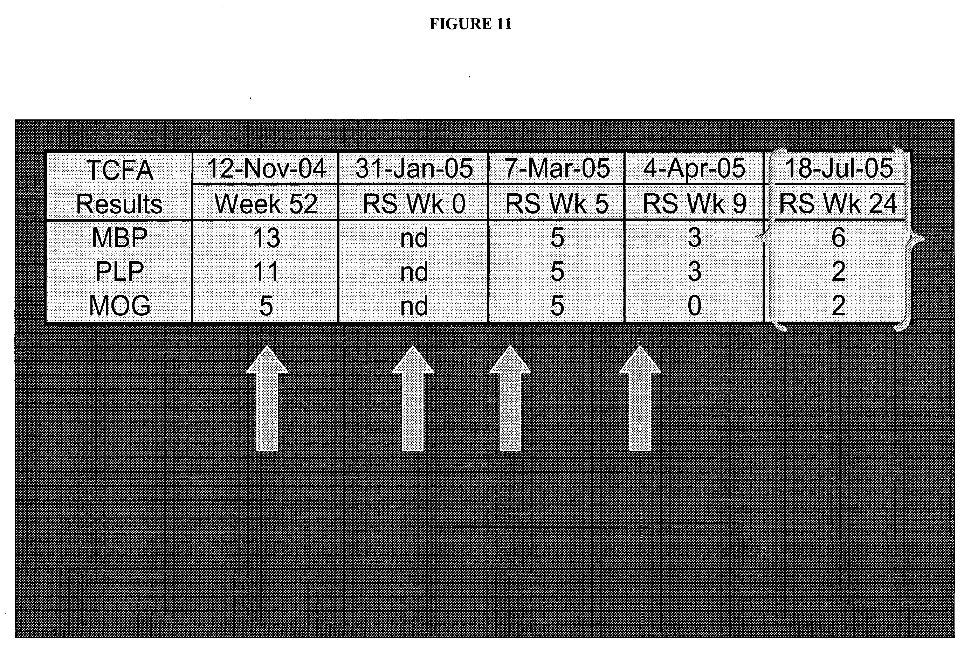

The EAA was used to evaluate myelin reactive T-cell (MRTC) specificity after T-cell vaccination. The detection of epitope spreading, which occurs when the initial, focused immune attack on the myelin proteins spreads to include new epitopes was also tested. Figure 11 shows some of the MRTC frequency data from a patient that had undergone the first retreatment series of 3 vaccines. At Week 52 following the initial series of vaccines, the patient's MRTCs had rebounded to a total of 29 MRTC per 10 million PBMCs. The TCFA prior, at Week 28, had shown only 4 MRTC/10 million PBMCs.

A new vaccine was prepared using the same procedures as the first series of vaccines and treatment was re-initiated. The patient received a booster at Week 4 and Week 8 and the MRTCs numbers dropped down to 6/10 million PBMCs. Fifteen weeks later, at Week 24, the patient's MRTC have started reappearing, particularly the MBP reactive T cells.

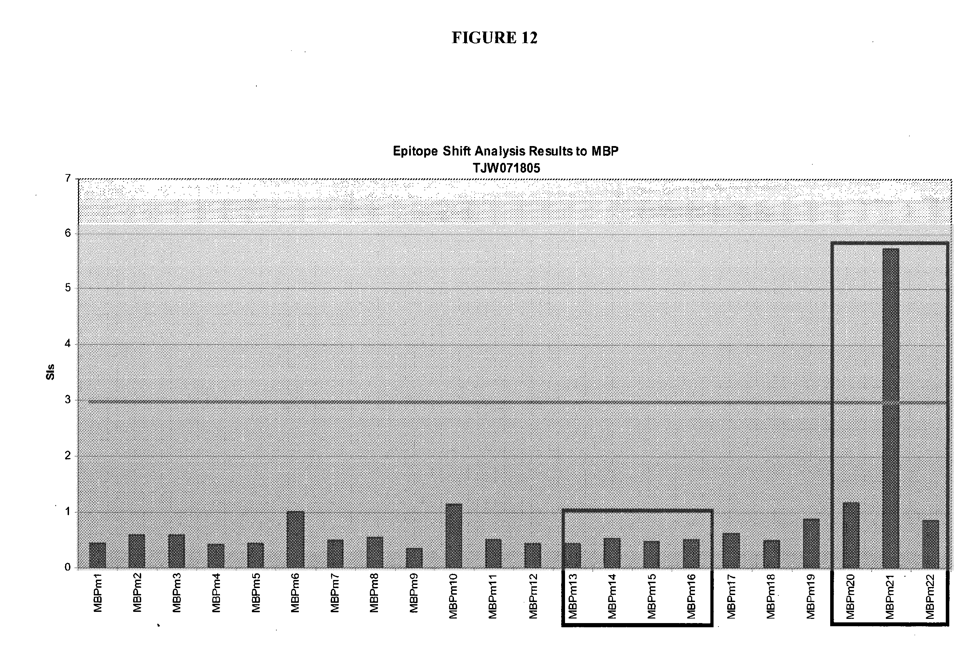

Figure 12 shows the reactivity of the patient's Week 24 T cells to the full length MBP peptides. The boxes surround the peptides that contain the two immunodominant MBP sequences used previously in the TCFA. As can be seen, there is still reactivity to MBP peptide 2, which may account for the increased reactivity against MBP also seen in the TCFA.

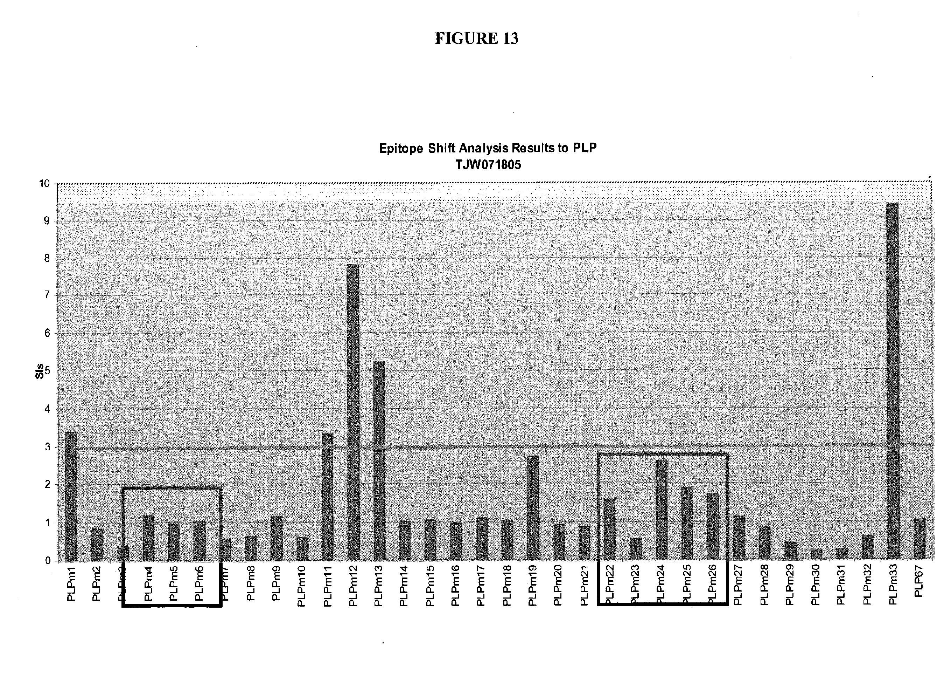

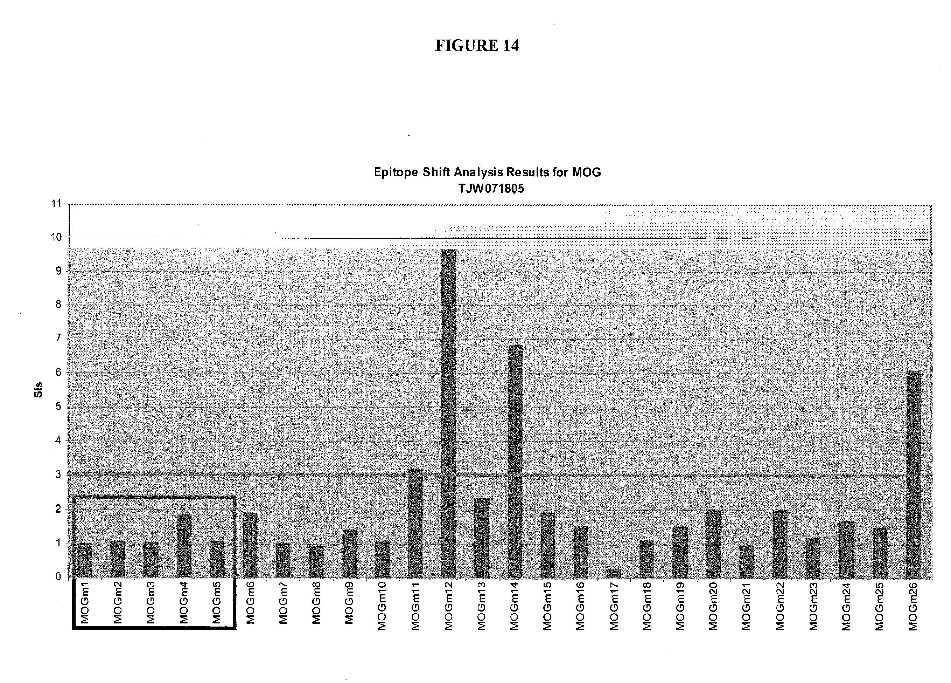

Figures 13 and 14 show the reactivity of the patient's week 24 T cells to the peptides spanning full length PLP and MOG proteins, respectively. The boxes surround the peptides that contain the two immunodominant PLP and MOG sequences used previously and in the TCFA. As indicated, there are three areas in PLP and two areas in MOG that show strong reactivity in the EAA. This analysis has been used to produce a new vaccine for the patient using the six newly identified reactive peptide mixes.

### Example 10

### Summary EAA Analysis 1

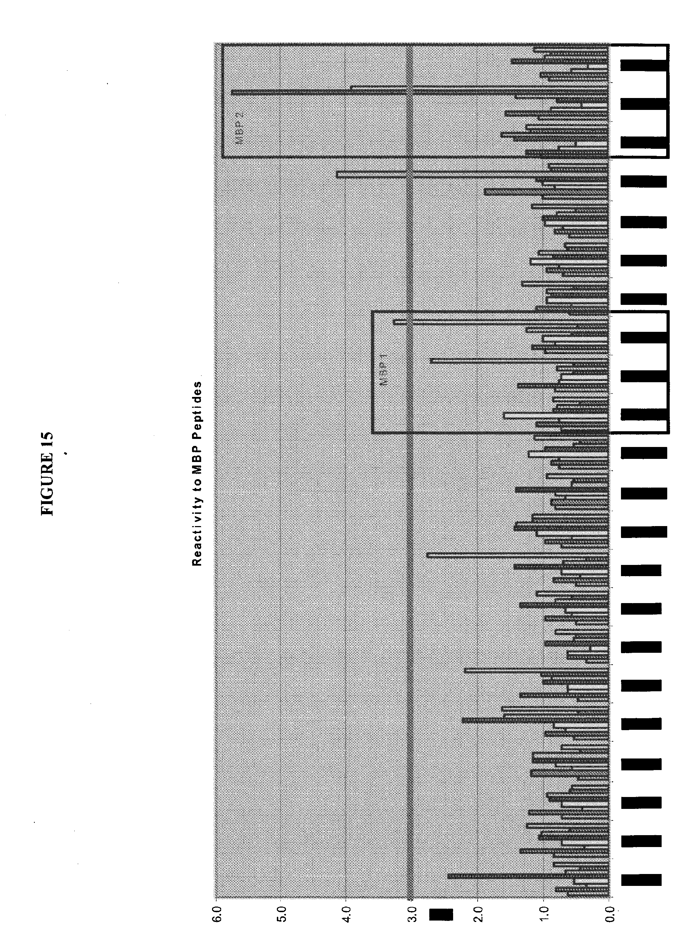

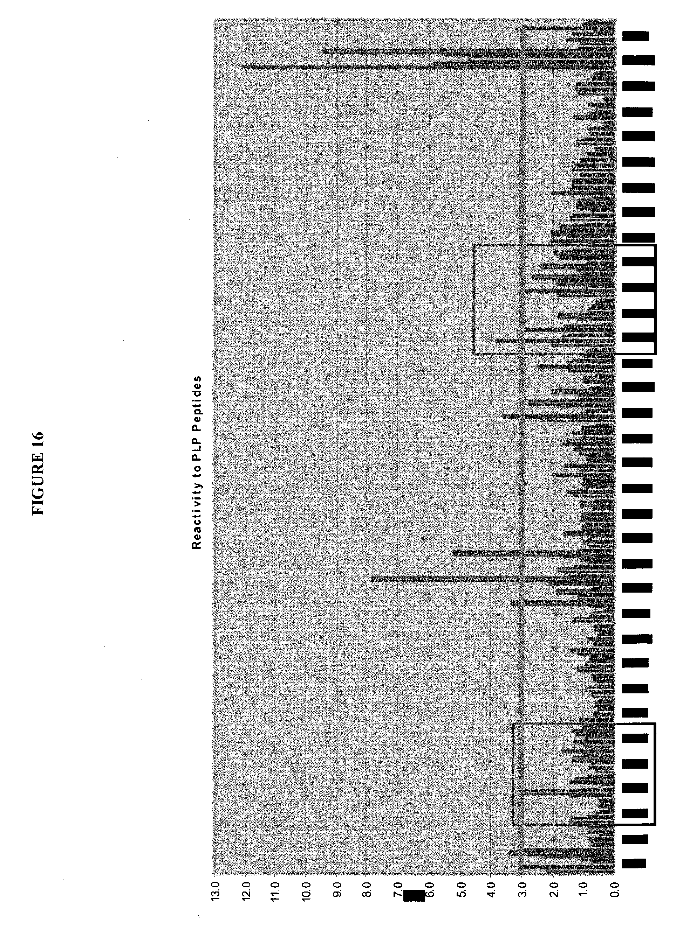

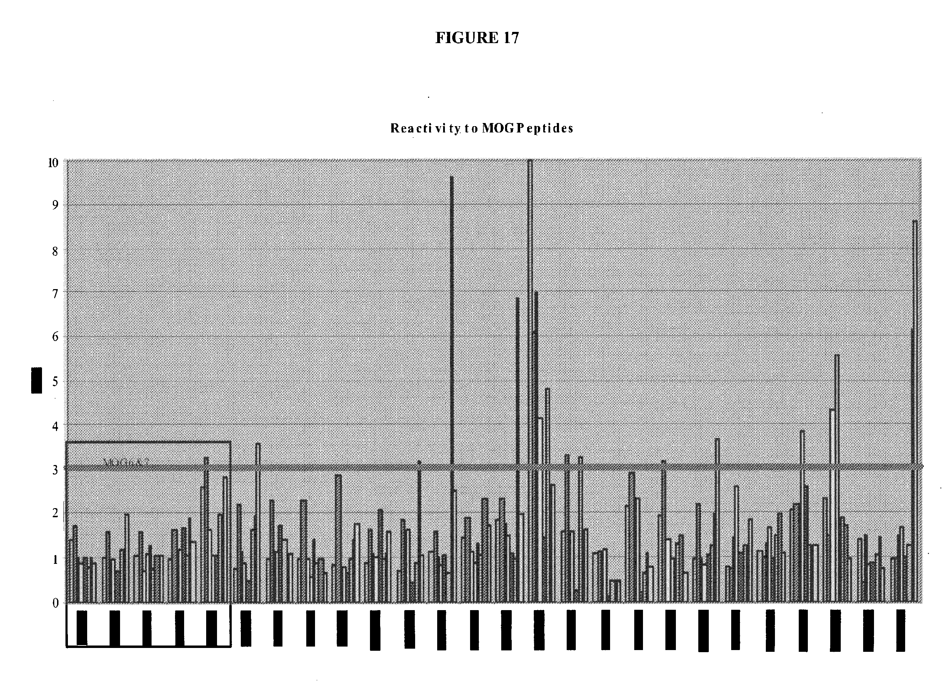

Figures 15-17 show the reactivity pattern to MBP, PLP and MOG peptides, respectively, in 8 MS patients. The red line shows the positive cut-off SI of 3. The two immunodominant peptides of MBP. PLP and MOG that were previously used are identified by the boxes. As indicated in Figure 15, there is an epitope within MBPm19 that is not present in the two immunodominant MBP peptides. For PLP, Figure 16 indicates that there are three other areas of immunoreactivity not included within the immunodominant PLP peptides. The highest immunoreactivity was within all 3 regions at the end of the PLP protein sequence. For MOG, Figure 17 indicates that the transmembrane and immediately intracellular portion of the protein is far more immunoreactive than the previously used immunodominant MOG peptides.

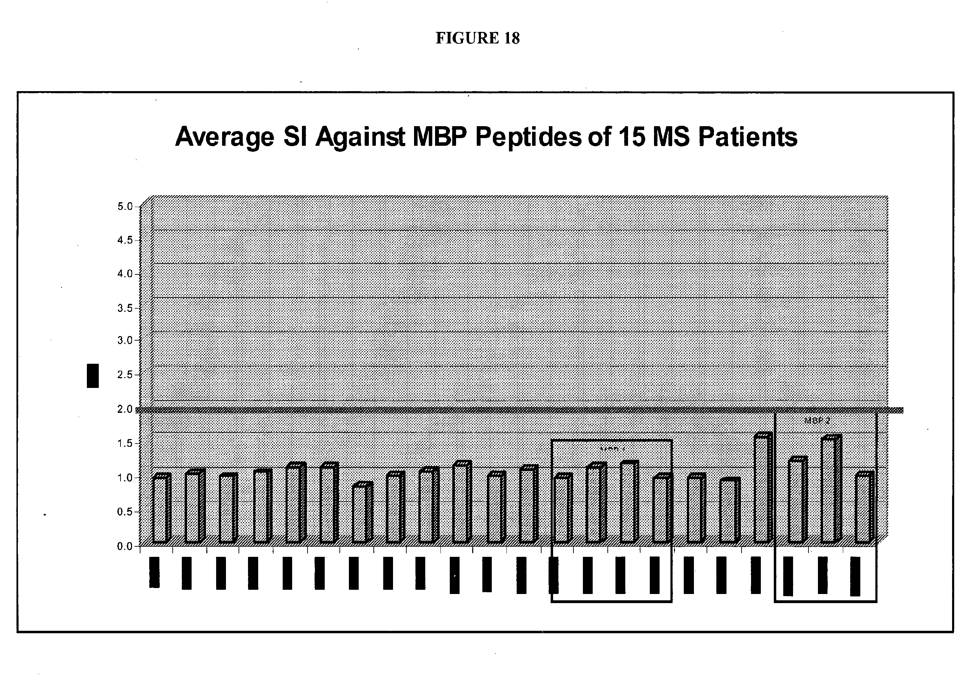

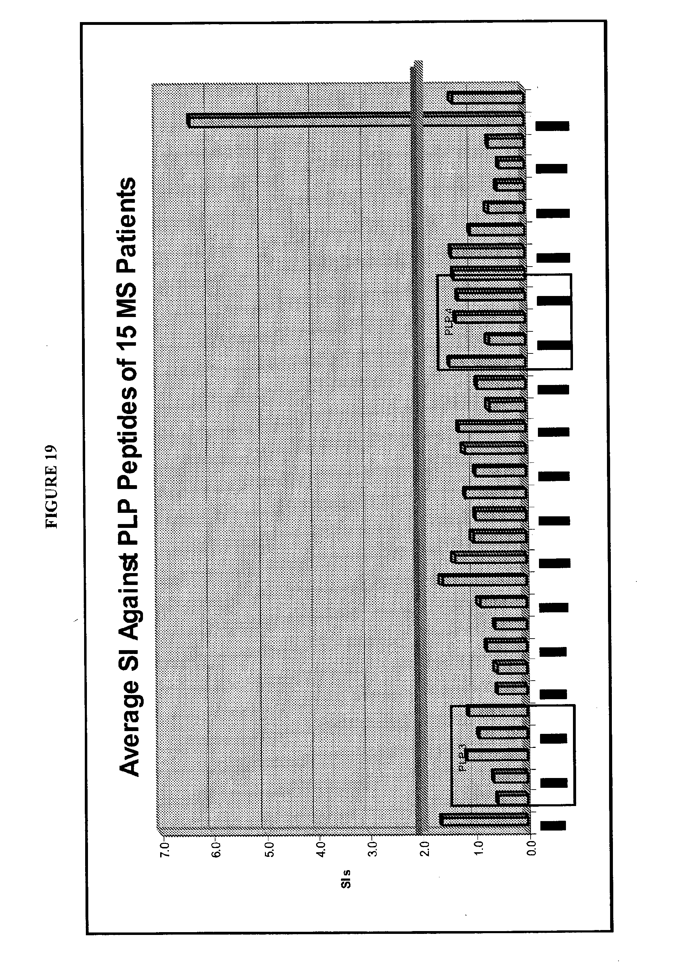

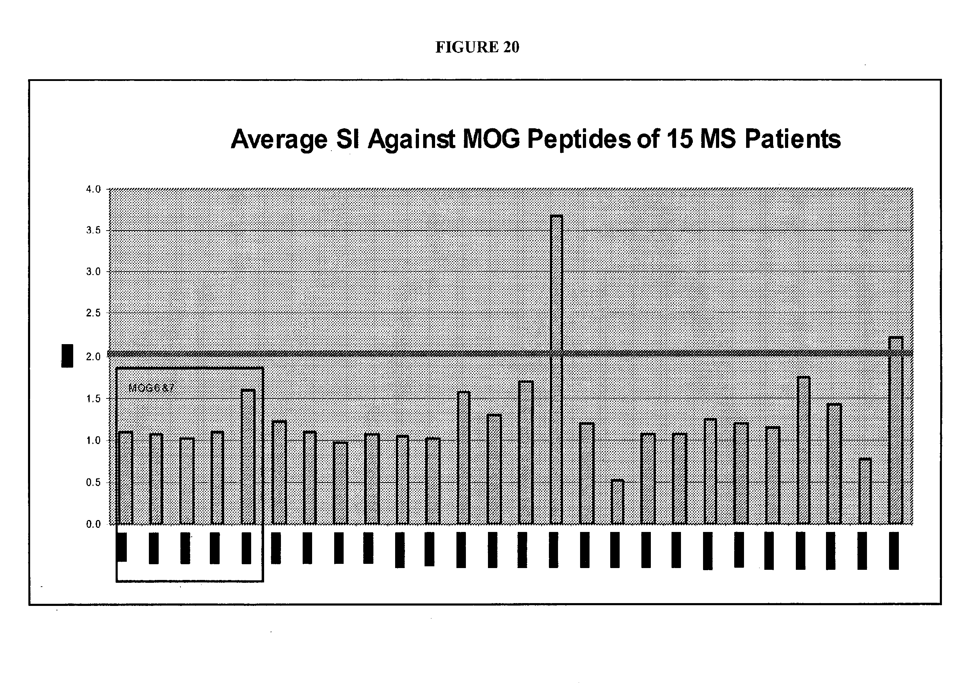

Figures 18-20 show the mean SIs for samples from a total of 15 MS patients tested using the EA assay. As can be seen, some slightly increased reactivity was seen toward the C-terminus of MBP. When the same data is analyzed for the PLP protein, the immunodominant area, again at the C-terminus of this protein, is very evident. Analysis of the reactivity against the MOG protein shows the immunodominant area at the transmembrane region as well as the C-terminus region of the protein. Taken together, the data indicate that it is important to include other peptide epitopes from within myelin proteins to identify myelin reactive T cells from MS patients for use in T-cell vaccines in order to produce a more effective vaccine in a more efficient manner.

Knowledge of the pattern of epitope spreading, or shift, in MS may be used to design peptide-specific T cell vaccination therapies that block ongoing tissue destruction. Sequential EAAs may be used to determine if and when a patient develops reactivity against "new" epitopes within the 3 myelin proteins analyzed. Newly identified reactive peptides may be used to produce T cell lines for a follow-on vaccine.

### Example 11

### Summary EAA Analysis 2

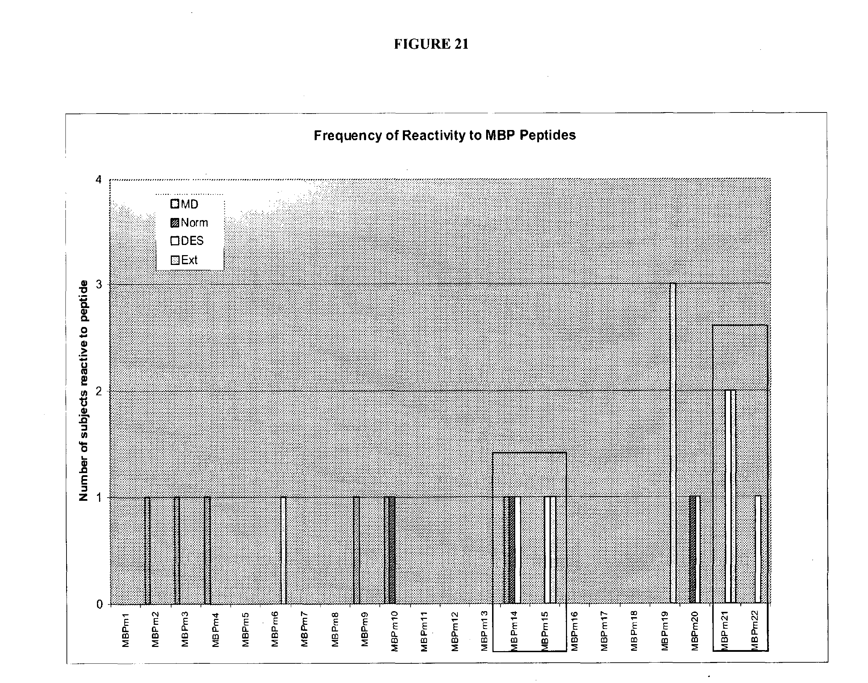

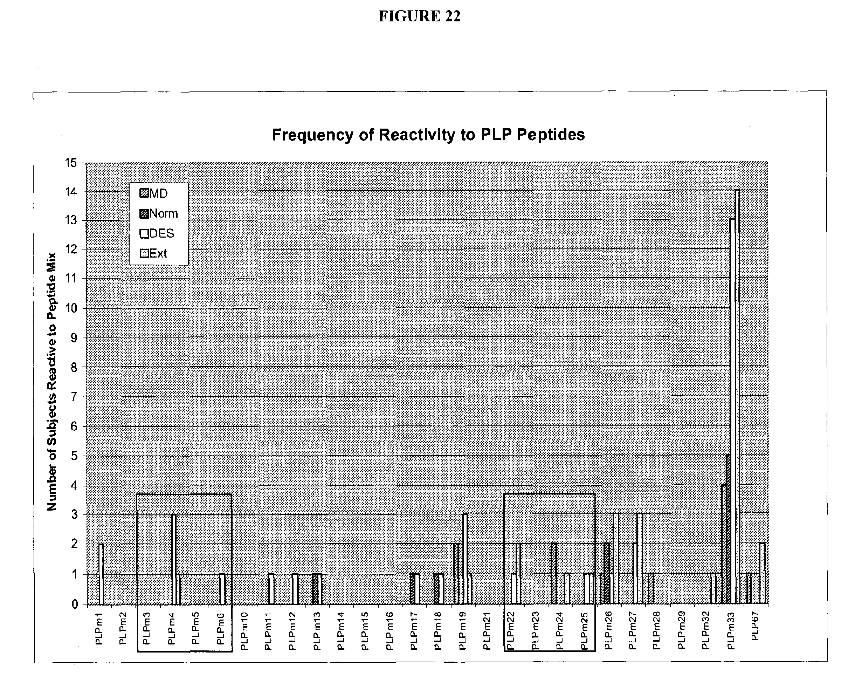

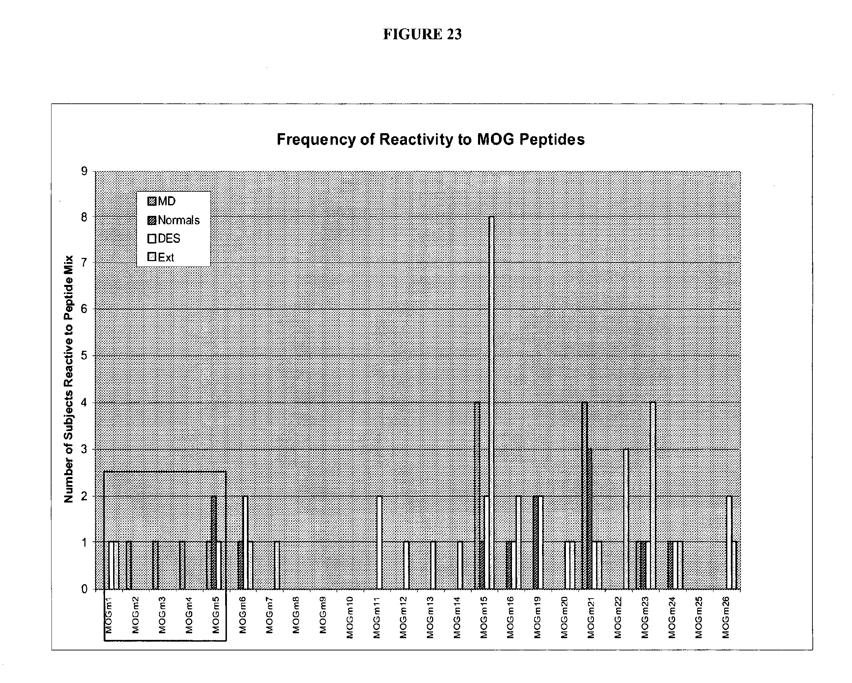

EAA was performed on 54 subjects. The subjects included healthy individuals (N=12), MS patients enrolled in a dose escalation clinical trial for Tovaxin (N=16), MS patients enrolled in a repeat vaccination (Extension Study) clinical trial for Tovaxin (N=13), and MS patients enrolled in a blood draw only (Method Development) clinical trial (N=13). Table 4 shows the reactivity of the subjects to the myelin peptide mixes. Tables 5-7 show the reactivity of the subjects to MBP, PLP and MOG peptide mixes, respectively. Figures 21-23 show the reactivity of the subjects to the myelin peptide mixes with the boxes indicating the location of the immunodominant peptides used in the production of the vaccine of Example 1.

**Table 7 Myelin Peptide Reactivity**

| Subject Group: | # reactive/N | Percent Reactive |
|---|---|---|
| Healthy | 7/12 | 58 |
| Method Development¹ | 9/13 | 69 |
| Dose Escalation | | |
| Vaccinated²: | 8/9 | 89 |
| Naive: | 5/7³ | 71 |
| Extension Study | | |
| Vaccinated²: | 9/9 | 100 |
| Naïve⁴: | 4/4 | 100 |

| | | |
|---|---|---|
| All 13 subjects tested were taking an approved immunomodulating MS therapy. ² Vaccinated with Tovaxin prepared using 6 peptides only. ³ The two samples that were negative were set up with frozen cells. ⁴ Naive to Tovaxin; these patients had been previously vaccinated with vaccine prepared with peptides from MBP only. | | |

**Table 8 MBP Peptide Reactivity**

| Subject Group: | # reactive/N | Percent Reactive |
|---|---|---|
| Healthy | 3/12 | 25 |
| Method Development | 3/13 | 23 |
| Dose Escalation | | |
| Vaccinated: | 2/9 | 22 |
| Naive: | 2/7 | 29 |
| Extension Study | | |
| Vaccinated: | 5/9 | 56 |
| Naive: | 1/4 | 25 |

**Table 9 PLP Peptide Reactivity**

| Subject Group: | # reactive/N | Percent Reactive |
|---|---|---|
| Healthy | 5/12 | 42 |
| Method Development Dose Escalation | 6/13 | 46 |
| Vaccinated: | 7/9 | 78 |
| Naive: | 4/7 | 57 |
| Extension Study | | |
| Vaccinated: | 8/9 | 89 |
| Naive: | 3/4 | 75 |

**Table 10 MOG Peptide Reactivity**

| Subject Group: | # reactive/N | Percent Reactive |
|---|---|---|
| Healthy | 6/12 | 50 |
| Method Development Dose Escalation | 7/13 | 54 |
| Vaccinated: | 4/9 | 44 |
| Naive: | 3/7 | 43 |
| Extension Study | | |
| Vaccinated: | 9/9 | 100 |
| Naive: | 3/4 | 75 |

Reactivity to at least one of the myelin peptide mixes was seen in 42 of the 54 subjects tested (78%), including 7 of the 12 healthy subjects. The number of peptide mixes that subjects reacted to ranged from 0 to 11. Positive reactivity ranged from the minimal SI of 3.0 to a maximal SI of 21.1.

As shown by the patterns of reactivity, the majority of the subjects tested had reactive T-cells to peptide sequences in areas of the three myelin proteins outside the six immunodominant peptides that were used to prepare vaccines in Example 1. Forty-one percent of the subjects tested were reactive to the immunodominant peptides of MBP (MBP 83-99 and MBP 151-170). Only 31% of subjects tested were reactive to the PLP immunodominant peptides (PLP 30-49 and PLP 180-199). Only 11% of subjects tested were reactive to the MOG immunodominant peptide sequences (MOG 1-17 and MOG 19-39).

The reactivity pattern initially appears consistent with previous studies identifying the peptide sequence MBP 83-99 as more reactive than PLP and MOG peptides. However, in general, the reactivity against MBP peptides was lower than the reactivity seen against PLP and MOG peptides; 30% of all subjects tested were reactive against one or more MBP peptides as opposed to 65% for PLP peptides and 61% for MOG peptides. Table 11 below shows the average number of peptides for each protein that the subjects were reactive against.

**Table 11**

| Average No. of Peptides Subjects are reactive against: | | | | |
|---|---|---|---|---|
| | MD (n=13) | H.S. (n=12 | DES (n=16) | EXT (n=13) |
| MBP | 0.5 | 0.3 | 0.4 | 0.5 |
| PLP | 0.8 | 0.8 | 2.0 | 2.2 |
| MOG | 1.0 | 1.0 | 1.3 | 1.8 |
| TOTALS: | 2.3 | 2.1 | 3.6 | 4.6 |

| | | | | |
|---|---|---|---|---|
| MD=Method Development Blood Draw subjects H.S.=Healthy Subjects DES=Dose Escalation (both vaccinated and naive) patients EXT=Extension Study patients | | | | |

In the case of the MOG protein, previous studies have focused on the extracellular portion of the protein. MOG has an extracellular part including aa 1-122 with an Ig-like domain, a transmembrane part, and an intracellular part comprising aa 123-218. The above results indicate a high level of reactivity to the portion of the protein that is within or immediately past the transmembrane sequence in the intracellular space, amino acids 113-132 (MOGm15). Interestingly, all (100%) of the Extension study patients, who had been previously vaccinated with the vaccine of Example 1, showed immunoreactivity to the intracellular portion of MOG. This is in contrast to all of the other subjects tested, of which only 49% showed any reactivity to MOG peptides.

### Growth of Myelin Reactive T Cell Lines with Low Stimulation Indices

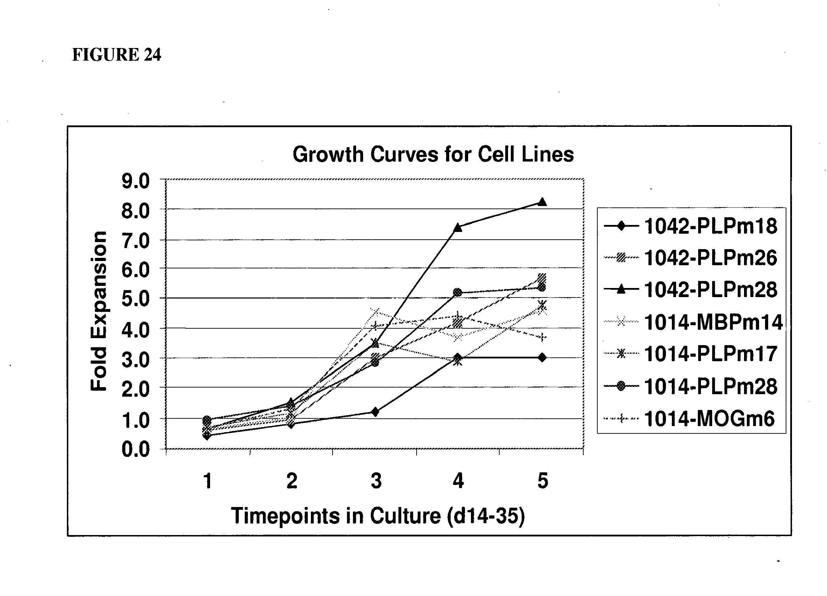

The following demonstrates the capability of myelin-reactive T cells to grow with an SI of less than 3.0 and especially below 2.0. Using the algorithms described above, statistically significant SIs as low as 1.3 have been observed and therefore should be able grow in response to antigen. To verify this ability, 5 cell lines across two patients are shown with an SI<2.0. The results are shown in Table 12 and Figure 24, which indicate growth of these cell lines with only peptide antigen stimulation and common T cell growth factors.

PBMC for subject 1042 were run in the EAA, and 2 peptide mixes were positive, including PLPm18 with an SI of 1.8, PLPm26 with an SI of 2.5 and PLPm28 with an SI of 2.5. Cells were subjected to antigenic stimulation as per normal protocol as shown below and collected 14, 19, 26, 33 and 35 days later. PBMC for subject 1014 were run in the EAA, and 4 mixes were positive, including MBPm14 with an SI of 1.7, PLPm17 with an SI of 1.7, PLPm28 with an SI of 2.2 and MOGm6 with an SI of 1.9. Cells for subject 1014 were subjected to antigenic stimulation as shown below and collected 14, 19, 26, 33 and 35 days later.

PBMCs were isolated via density gradient centrifugation from peripheral blood obtained through venipuncture into ACD-1 anticoagulant. PBMCs were plated at 2.5E+06 cells/well in 24 well plates. Antigen in the form of 16mer peptides identified in the EAA were added at a final concentration of 20ug/ml. Interluekin-2 (IL-2) was added at a final concentration of 100U/ml starting at 48 hours and IL-2 was added at the same concentration with each feeding or splitting of wells. Peptide restimulation in the presence of antigen presenting cells (APCs-autologous PBMCs irradiated at 3500rad) was done at days 7, 14 and 21. Interleukin-15 (IL-15) was added at a final concentration of 5ng/ml-20ng/ml (lot specific) starting at day 14 and continued through the remainder of the culture period. Cells lines were all harvested by day 35 and had achieved expansions of 3.0-8.2 fold.

**Table 12**

| **Subject** | **Peptide Mix** | **Viable Cell Counts** | **Viable Cell Count** | | | | | |
|---|---|---|---|---|---|---|---|---|
| | | | **Day 0** | **Day 14** | **Day 19** | **Day 26** | **Day 33** | **Day 35** |
| 1042 | PLPm18 (SI 1.9) | Count/well | 2.50E+06 | 9.60E+05 | 1.97E+06 | 1.50E+06 | 3.76E+06 | 3.74E+06 |
| | | No. of wells | 12 | 12 | 12 | 24 | 24 | 24 |
| | | Total Cells | 3.00E+07 | 1.15E+07 | 2.36E+07 | 3.60E+07 | 9.02E+07 | 8.98E+07 |
| | | Fold Expansion | 1.0 | 0.4 | 0.8 | 1.2 | 3.0 | 3.0 |
| | PLPm26 (SI 2.5) | Count/well | 2.50E+06 | 1.50E+06 | 2.40E+06 | 3.73E+06 | 2.61 E+06 | 3.54E+06 |
| | | No. of wells | 12 | 12 | 12 | 24 | 48 | 48 |
| | | Total Cells | 3.00E+07 | 1.80E+07 | 2.88E+07 | 8.95E+07 | 1.25E+08 | 1.70E+08 |
| | | Fold Expansion | 1.0 | 0.6 | 1.0 | 3.0 | 4.2 | 5.7 |
| | PLPm28 (SI 2.5) | Count/well | 2.50E+06 | 1.56E+06 | 3.82E+06 | 4.34E+06 | 4.61 E+06 | 5.15E+06 |
| | | No. of wells | 12 | 12 | 12 | 24 | 48 | 48 |
| | | Total Cells | 3.00E+07 | 1.87E+07 | 4.58 E+07 | 1.04E+08 | 2.2 E+08 | 2.47E+08 |
| | | Fold Expansion | 1.0 | 0.6 | 1.5 | 3.5 | 7.4 | 8.2 |

| **Subject** | **Peptide Mix** | **Viable Cell Counts** | **Viable Cell Count** | | | | | |
|---|---|---|---|---|---|---|---|---|
| | | | **Day 0** | **Day 17** | **Day 19** | **Day 28** | **Day 33** | **Day 35** |
| 1014 | MBPm14 (SI 1.7) | Count/well | 2.50E+06 | 2.04E+06 | 3.41 E+06 | 4.38E+06 | 3.54E+06 | 4.42E+06 |
| | | No. of wells | 9 | 9 | 9 | 31 | 31 | 31 |
| | | Total Cells | 2.25E+07 | 1.84E+07 | 3.07E+07 | 1.36E+08 | 1.10E+08 | 1.37E+08 |
| | | Fold Expansion | 1.0 | 0.6 | 1.0 | 4.5 | 3.7 | 4.6 |
| | PLPm17 (SI 1.7) | Count/well | 2.50E+06 | 2.34E+06 | 3.85E+06 | 4.77E+06 | 3.89E+06 | 4.73E+06 |
| | | No. of wells | 9 | 9 | 9 | 22 | 22 | 30 |
| | | Total Cells | 2.25E+07 | 2.11E+07 | 3.47E+07 | 1.05E+08 | 8.56E+07 | 1.42E+08 |
| | | Fold Expansion | 1.0 | 0.7 | 1.2 | 3.5 | 2.9 | 4.7 |
| | PLPm28 (SI 2.2) | Count/well | 2.50E+06 | 3.09E+06 | 2.41 E+06 | 2.81E+06 | 5.17E+06 | 5.33E+06 |
| | | No. of wells | 9 | 9 | 18 | 30 | 30 | 30 |
| | | Total Cells | 2.25E+07 | 2.78E+07 | 4.34E+07 | 8.43E+07 | 1.55E+08 | 1.60E+08 |
| | | Fold Expansion | 1.0 | 0.9 | 1.4 | 2.8 | 5.2 | 5.3 |
| | MOGm6 (SI 1.9) | Count/well | 2.50E+06 | 2.18E+06 | 4.27E+06 | 4.35E+06 | 4.71 E+06 | 3.93E+06 |
| | | No. of wells | 12 | 9 | 9 | 28 | 28 | 28 |
| | | Total Cells | 3.00E+07 | 1.96E+07 | 3.84E+07 | 1.22E+08 | 1.32E+08 | 1.10E+08 |
| | | Fold Expansion | 1.3 | 0.7 | 1.3 | 4.1 | 4.4 | 3.7 |

Figure 24 shows that the 5 T cell lines from subjects 1042 and 1014 reactive to myelin peptide mixes with SIs < 2.0 were capable of growing in response to antigen. Therefore T cell lines exhibiting low SIs can be grown in response to a myelin antigen.

### Example 12

### EAA Analysis of Myelin Reactive Peptides

The peptides of Example 3 have been tested in 429 EAAs in clinical trials as follows. A total of 162 out of 429 assays (37.8%) showed positive SIs using the Variance Evaluation Method or CPM Variance Method. Pre-Vaccine EAAs have 158 positive out of 387 assays (40.8%), with Relapse Remitting MS patients (RRMS) showing 150 positive of 368 assays (40.8%), and Clinically Isolated Syndrome (CIS) patients showing 8 positive of 19 assays (42.1%).

Of screened subjects, 144 assays out of 312 total have been positive from 249 subjects (46.2% and 57.8% respectively). Of these, RRMS patients showed 136 positive out of 301 assays on 238 subjects (45.2% and 57.1%, respectively), and CIS patients showed 8 positive out of 11 assays (72.7%).

During procurement 14 of 89 assays (15.7%) have been positive, of which RRMS patients have shown 14 positive of 84 assays (16.7%), and CIS patients showed 0 positive out of 5 assays (0%). During baseline EAAs, 6 of 31 assays (19.4%) have been positive, with RRMS patients showing 6 positive out of 28 assays (21.4%), CIS patients showing 0 positive of 3 assays (0%).

Post-vaccine EAAs have shown 4 out of 42 assays (9.5%) positive, of which RRMS patients showed 3 positive out of 37 assays (8.1 %), and CIS patients showed 1 positive out of 5 assays (20.0%).

Week 4 EEAs have shown 2 positive out of 21 assays (9.5%), of which RRMS patients showed 1 positive out of 19 assays (5.3%), and CIS patients have shown 1 positive out of 2 assays (50.0%). Week 8 EAAs showed 2 positive out of 16 assays (12.5%), of which RRMS patients showed 2 positive out of 14 assays (14.3%) and CIS patients showed 0 positive of 2 assays (0%). Week 12 EAAs showed 0 positive of 5 assays (0%), of which RRMS patients showed 0 positive of 4 assays (0%) and CIS showed 0 positive of 1 assay (0%).

### Example 13

### EAA Clinical Trial

Over the course of 6 months a study was conducted with 120 subjects meeting the criteria for the clinical trial. Subjects had Relapse Remitting Multiple Sclerosis (RR-MS; n=114) or high risk Clinically Isolated Syndrome (CIS; n=6) with an Expanded Disability Status Scale (EDSS) of 0-5.5, diagnosis of disease of 0-10 years, 18-55 years of age, MRI criteria suggestive of MS and a positive EAA.

The above mentioned population of subjects was evaluated by EAA using the Variance Evaluation Method and CPM Variance Method as described above, and deemed eligible for vaccine production. Of the 120 attempts to manufacture vaccine, there were 2 shipping failures (blood was not received within our stated criteria), 1 culture was lost to contamination and 5 samples arrived with low cell volume/yield. Of the remaining 112 subjects, vaccine was successfully generated in 89 subjects, with 22 still in production (98.9% of eligible samples, 92.5% of all samples completed). From this, a positive EAA was deemed predictive of the ability to manufacture a vaccine to therapeutic dose by methods disclosed herein.

Current methods to grow T cell lines include:
1. Isolation of PBMCs
2. Plating of PBMCs in 24 well plates at 2.5E+06 cells/well
3. Adding antigen at 20ug/ml/peptides, 2 peptides/well
4. Addition of IL-2 (10-200 IU/ml) at 48 hours (and included until harvest)
5. Restimulations with APCs irradiated at 3500 rads (1.0E+06 as described before) and antigen (same antigen/same concentration) at days 7, 14 and 21
6. Addition of IL-15 (1-50 ng/ml) at day 14 (and included until harvest)
7. Potential for addition of mitogen, superantigen or antibody to stimulate growth at d35 if the cells have not grown to sufficient quantity

Over the course of this study several epitopes with immunodominance were discovered in this population of MS subjects. Several of these epitopes are novel. These are peptides showed reactivity in a large number of subjects screened in our trial. Several of these immunodominant regions have not previously been described in the literature. The epitopes of interest are listed in Tables 13-15.

**Table 13 Listed by frequency of identification**

| **Peptide Mix** | **# of Subjects** | **% of Subjects** |
|---|---|---|
| **MOGm21** | **40** | **33.6%** |
| **PLPm26** | **28** | **23.5%** |
| **MOGm15** | **27** | **22.7%** |
| **PLPm4** | **26** | **21.8%** |
| **MOGm23** | **20** | **16.8%** |
| **MOGm24** | **15** | **12.6%** |
| **PLPm17** | **14** | **11.8%** |
| **PLPm32** | **12** | **10.1%** |
| **PLPm19** | **11** | **9.2%** |
| **PLPm33** | **11** | **9.2%** |
| **PLPm18** | **10** | **8.4%** |
| MOGm26 | 9 | 7.6% |
| MBPm8 | 8 | 6.7% |
| MOGm5 | 8 | 6.7% |
| MBPm9 | 7 | 5.9% |
| PLPm24 | 7 | 5.9% |
| MBPm13 | 6 | 5.0% |
| MOGm19 | 6 | 5.0% |
| MOGm2 | 6 | 5.0% |
| PLPm27 | 6 | 5.0% |
| PLPm28 | 6 | 5.0% |
| MBPm14 | 5 | 4.2% |
| MBPm20 | 5 | 4.2% |
| MBPm3 | 5 | 4.2% |
| MOGm11 | 5 | 4.2% |
| MOGm7 | 5 | 4.2% |
| PLP67 | 5 | 4.2% |
| MBPm16 | 4 | 3.4% |
| MBPm17 | 4 | 3.4% |
| MBPm22 | 4 | 3.4% |
| MOGm12 | 4 | 3.4% |
| MOGm16 | 4 | 3.4% |
| MOGm3 | 4 | 3.4% |
| MOGm6 | 4 | 3.4% |
| PLPm1 | 4 | 3.4% |
| PLPm11 | 4 | 3.4% |
| PLPm6 | 4 | 3.4% |
| MBPm15 | 3 | 2.5% |
| MBPm19 | 3 | 2.5% |
| MBPm2 | 3 | 2.5% |
| MBPm4 | 3 | 2.5% |
| MBPm7 | 3 | 2.5% |
| MOGm1 | 3 | 2.5% |
| MOGm4 | 3 | 2.5% |
| PLPm13 | 3 | 2.5% |
| PLPm25 | 3 | 2.5% |
| MBPm10 | 2 | 1.7% |
| MBPm12 | 2 | 1.7% |
| MOGm14 | 2 | 1.7% |
| MOGm22 | 2 | 1.7% |
| PLPm22 | 2 | 1.7% |
| MBPm21 | 1 | 0.8% |
| MBPm6 | 1 | 0.8% |
| MOGm20 | 1 | 0.8% |
| PLPm12 | 1 | 0.8% |

**Table 14 Peptide mixes positive in >8% of this population**

| **Peptide Mix** | **N term** | | **C term** | **Amino Acid Sequences** |
|---|---|---|---|---|
| PLPm4 | 25 | to | 44 | see Example 2 |
| PLPm17 | 129 | to | 148 | |
| PLPm18 | 137 | to | 156 | |
| PLPm19 | 145 | to | 160 | |
| PLPm26 | 201 | to | 220 | |
| PLPm32 | 249 | to | 268 | |
| PLPm33 | 257 | to | 276 | |
| MOGm15 | 113 | to | 132 | |
| MOGm21 | 161 | to | 180 | |
| MOGm23 | 177 | to | 196 | |
| MOGm24 | 185 | to | 204 | |

**Table 15 Listed as immunodominant sequences**

| **Protein** | **Peptide Stretch** | | | **Amino Acid Sequences** |
|---|---|---|---|---|
| PLP | 25 | to | 44 | see Example 2 |
| PLP | 129 | to | 160 | |
| PLP | 201 | to | 220 | |
| PLP | 249 | to | 276 | |
| *MOG | 113 | to | 132 | |
| MOG | 161 | to | 204 | |

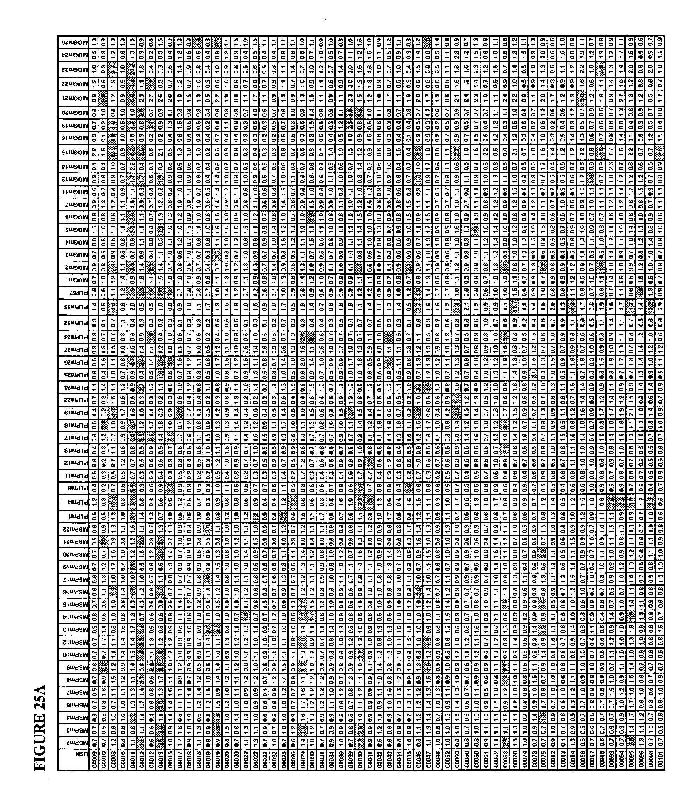

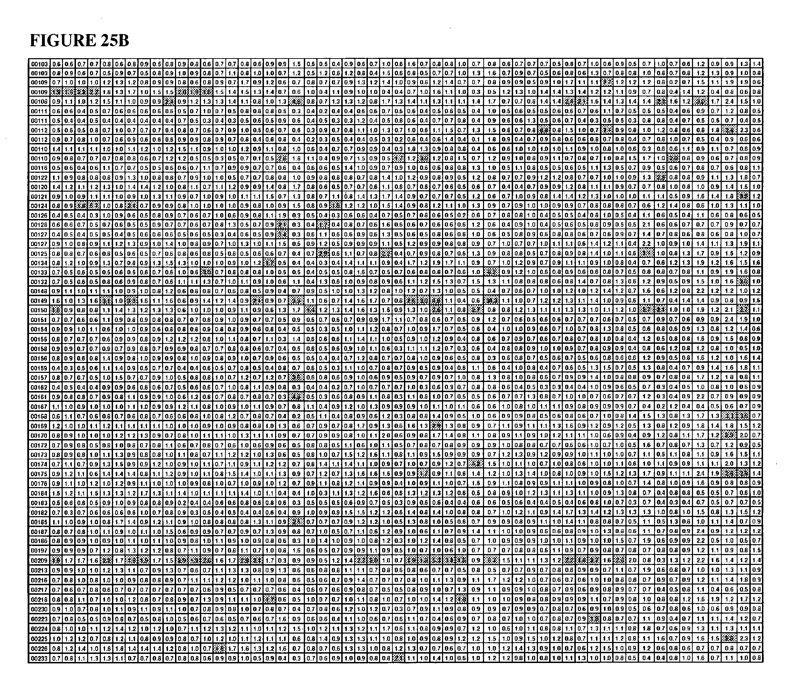

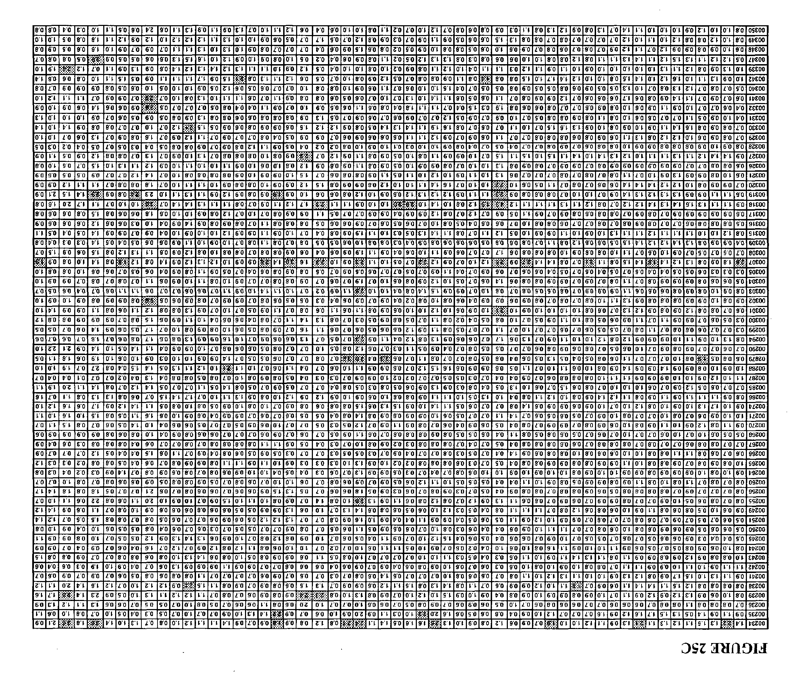

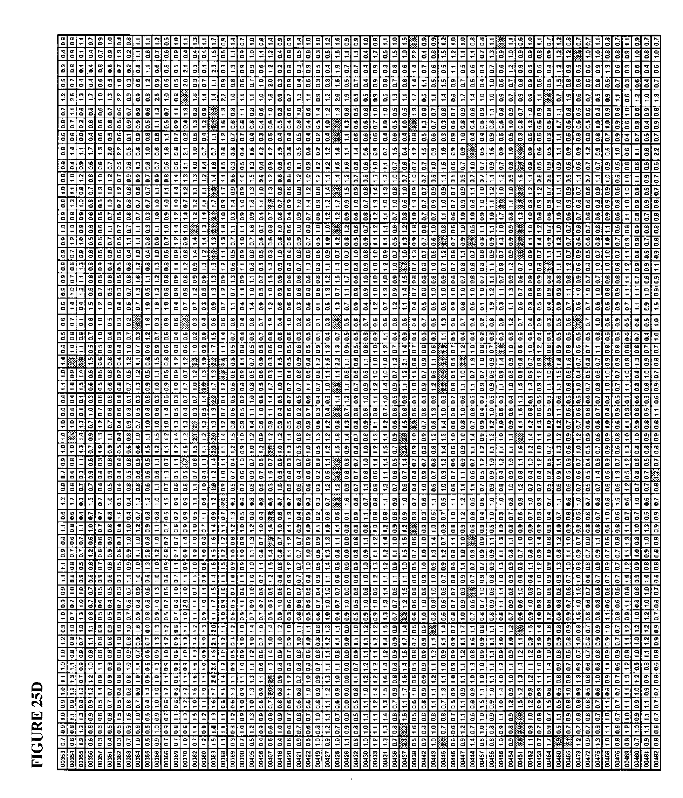

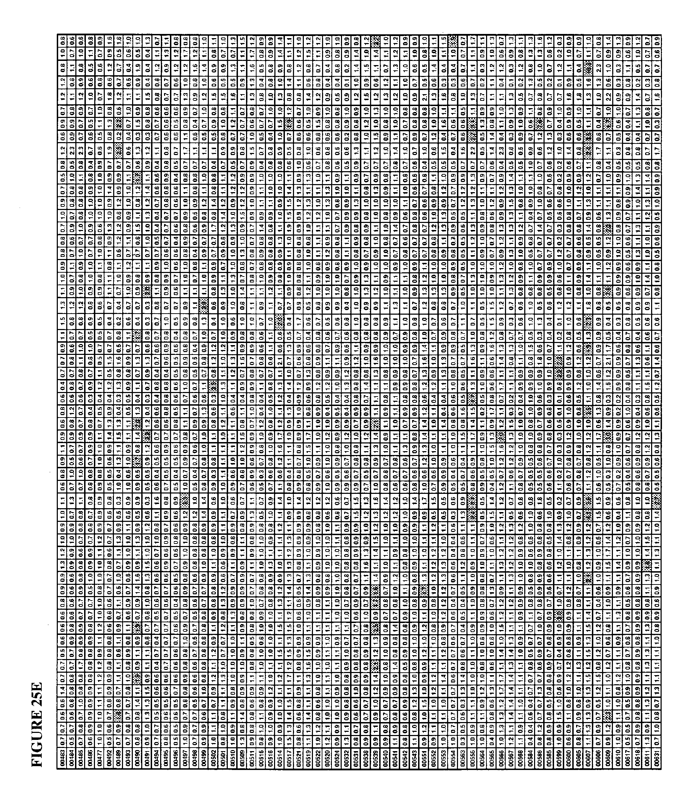

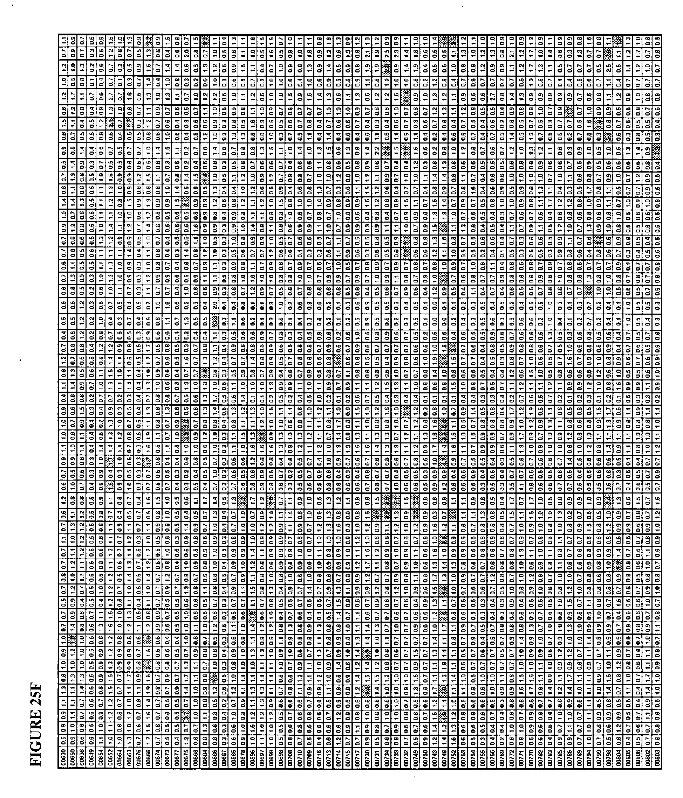

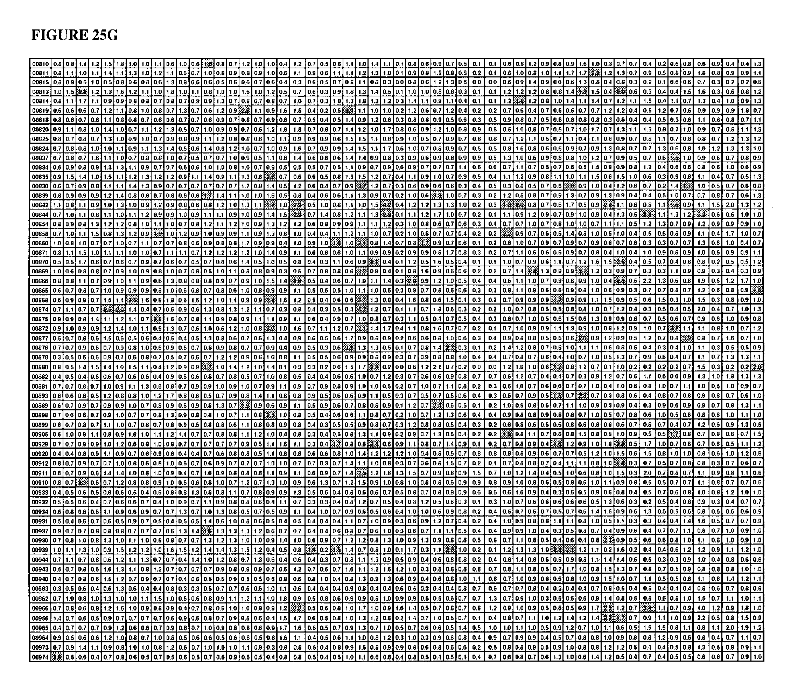

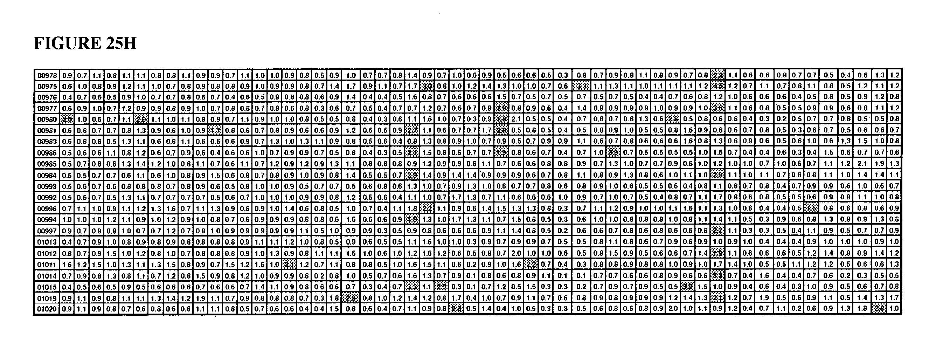

Figures 25-27 shows the results of this clinical trial. Figures 25A-H show a map of 429 assay (unique assays running down and peptides running across). Assays are aligned by date. Positive peptide mixes are shown in grey. This shows that the peptides mixes found to be positive were unique and unpredictable.

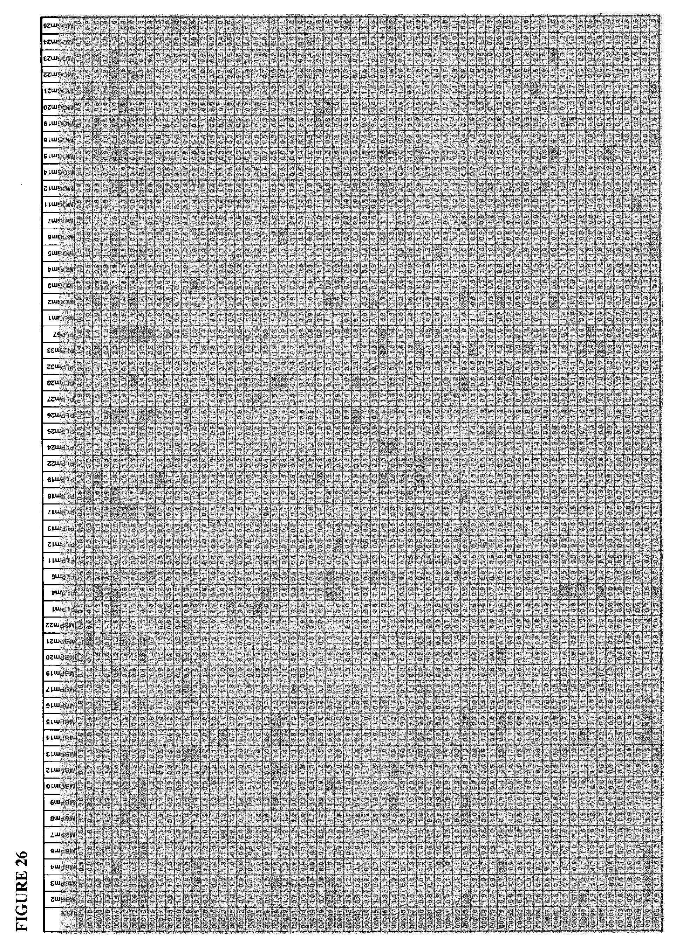

Figure 26 shows a map of 65 assay (unique assays running down and peptides running across). Assays are aligned by date. Positive peptide mixes are shown in grey. This shows that the peptides mixes found to be positive were unique and unpredictable. The number of positive mixes ranged in a single subject from 0 to 19.

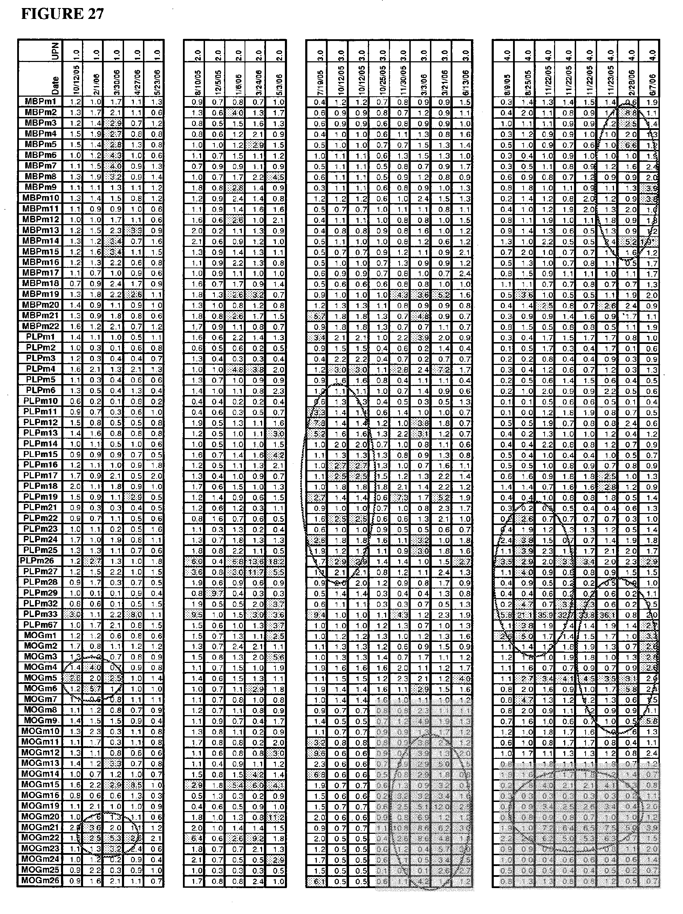

Figure 27 shows the results of EAA analysis indicating epitope shift over time in 4 subjects. Subject 1 showed a small shift with reactivity first around MOGm4, MOGm5 and MOGm6 and moving to MOGm21, MOGm22 and MOGm23. Notice that these two overlap. Subject 2 showed the same peptides reactive over time, but with increasing reactivity to new peptides the further from time zero. Subject 3 showed a significant shift in reactivity from PLP to MOG and subject 4 showed multiple epicenters for reactivity with a gradual shift from one area (C-terminal PLP) to another (N-terminal MOG). Also in this same subject a long term but transient reactivity to the central portion of MOG and finally a reactivity to MBP at the last two timepoints was observed.

## Claims

1. A method of preparing a T cell vaccine, comprising:
(a) providing a sample comprising T cells isolated from a patient;
(b) adding peptide mixes each comprising different peptides to a plurality of portions of the sample, wherein each different peptide in a peptide mix is from 8-20 amino acids and comprises an overlapping sequence of 4-19 amino acids with another different peptide in the peptide mix; and wherein the sequences of the different peptides in the peptide mixes collectively comprise the complete sequence of a polypeptide antigen; and
(c) identifying a portion of the sample comprising activated autoreactive T cells,
wherein a peptide mix that activates the autoreactive cells identified by (c) with a stimulation index above a predetermined value is a patient-specific peptide mix;
(d) providing a further sample comprising T cells isolated from the patient;
(e) contacting the T-cells with the patient-specific peptide mixes identified in steps (a) to (c), whereby autoreactive T-cells are activated;
(f) expanding activated autoreactive T cells; and
(g) attenuating the autoreactive T cells, so that the T cells are replication incompetent yet viable;
wherein the stimulation index for each peptide mix is calculated by comparing [³H]thymidine incorporation in a sample of the patient's T cells cultured in the presence of a peptide mix comprising a portion of the polypeptide to [³H]thymidine incorporation in a sample of the T cells cultured in a media only control, expressed as the quotient of the mean counts per minute of antigen aliquots / mean counts per minute of control aliquots, wherein the predetermined value is at least 1.3.

2. The method of claim 1 wherein the polypeptide antigen is MBP, PLP, MOG, or a combination thereof.

3. The method of claim 1 wherein the different peptides are 12-16 amino acids and comprise overlapping sequences of 8-12 amino acids.

## Patentansprüche

1. Verfahren zur Herstellung einer T-Zell-Vakzine, das Folgendes umfasst:
(a) die Bereitstellung einer Probe, die aus einem Patienten isolierte T-Zellen umfasst;
(b) das Zusetzen von Peptidgemischen, die jeweils unterschiedliche Peptide umfassen, zu einer Vielzahl von Teilen der Probe, worin jedes unterschiedliche Peptid in einem Peptidgemisch 8 bis 20 Aminosäuren lang ist und eine überlappende Sequenz in der Länge von 4 bis 19 Aminosäuren mit einem anderen unterschiedlichen Peptid in dem Peptidgemisch umfasst und worin die Sequenzen der unterschiedlichen Peptide in den Peptidgemischen kollektiv die vollständige Sequenz eines Polypeptid-Antigens umfassen;
und
(c) das Identifizieren eines Teils der Probe, der aktivierte autoreaktive T-Zellen umfasst,
worin ein Peptidgemisch, das die in (c) identifizierten autoreaktiven Zellen mit einem Stimulationsindex oberhalb eines vorbestimmten Werts aktiviert, ein patientenspezifisches Peptidgemisch ist;
(d) das Bereitstellen einer weiteren Probe, die aus dem Patienten isolierte T-Zellen umfasst;
(e) das Kontaktieren der T-Zellen mit in den Schritten (a) bis (c) identifizierten Peptidgemischen, wodurch autoreaktive T-Zellen aktiviert werden;
(f) das Expandieren der aktivierten autoreaktiven T-Zellen und
(g) das Attenuieren der autoreaktiven T-Zellen, so dass diese nicht zur Replikation in der Lage, aber doch lebensfähig sind;
worin der Stimulationsindex für jedes Peptidgemisch durch einen Vergleich der Inkorporation von [³H]-Thymidin in eine Probe von in Gegenwart eines Peptidgemischs, das einen Teil des Polypeptids umfasst, kultivierten T-Zellen des Patienten mit der Inkorporation von [³H]-Thymidin in eine Probe von in einer nur Medium enthaltenden Kontrolle kultivierten T-Zellen berechnet wird und als Quotient der mittleren Counts pro Minute von Antigenaliquoten und der mittleren Counts pro Minute von Kontrollaliquoten ausgedrückt wird, worin der vorbestimmte Wert zumindest 1,3 beträgt.

2. Verfahren nach Anspruch 1, worin das Polypeptid-Antigen MBP, PLP, MOG oder eine Kombination davon ist.

3. Verfahren nach Anspruch 1, worin die unterschiedlichen Peptide 12 bis 16 Aminosäuren lang sind und überlappende Sequenzen in einer Länge von 8 bis 12 Aminosäuren umfassen.

## Revendications

1. Procédé de préparation d'un vaccin à lymphocytes T, comprenant :
(a) la mise à disposition d'un échantillon comprenant des lymphocytes T isolés chez un patient ;
(b) l'ajout de mélanges de peptides, chacun comprenant des peptides différents par rapport à une pluralité de parties de l'échantillon, où chaque peptide différent dans un mélange de peptides possède entre 8-20 acides aminés et comprend une séquence chevauchante de 4-19 acides aminés avec un autre peptide différent dans le mélange de peptides ; et où les séquences des peptides différents dans les mélanges de peptides comprennent collectivement la séquence complète d'un antigène polypeptidique ; et
(c) l'identification d'une partie de l'échantillon comprenant des lymphocytes T auto-réactifs activés,
où un mélange de peptides qui active les cellules auto-réactives identifiées par (c) avec un indice de stimulation supérieur à une valeur prédéterminée est un mélange de peptides spécifique à un patient ;
(d) la mise à disposition d'un échantillon supplémentaire comprenant des lymphocytes T isolés chez le patient ;
(e) la mise en contact des lymphocytes T avec les mélanges de peptides spécifiques à un patient identifiés aux étapes (a) à (c), moyennant quoi des lymphocytes T auto-réactifs sont activés ;
(f) l'expansion des lymphocytes T auto-réactifs activés ; et
(g) l'atténuation des lymphocytes T auto-réactifs, de sorte que les lymphocytes T soient incompétents en réplication tout en étant viables ;
où l'indice de stimulation pour chaque mélange de peptides est calculé par une comparaison de l'incorporation de thymidine-[H³] dans un échantillon des lymphocytes T du patient mis en culture en présence d'un mélange de peptides comprenant une partie du polypeptide avec l'incorporation de thymidine-[H³] dans un échantillon des lymphocytes T mis en culture dans un milieu de contrôle uniquement, exprimé en tant que quotient des coups par minute moyens des aliquotes d'antigène/coups par minute moyens des aliquotes de contrôle, où la valeur prédéterminée est au moins 1,3.

2. Procédé selon la revendication 1, dans lequel l'antigène polypeptidique est la MBP, PLP, MOG, ou une combinaison de celles-ci.

3. Procédé selon la revendication 1, dans lequel les peptides différents possèdent 12-16 acides aminés et comprennent des séquences chevauchantes de 8-12 acides aminés.


### SEQUENCE LISTING

<110> OPEXA THERAPEUTICS Williams, Jim C. Montgomery, Mitzi Newsom, Brian
<120> T-CELL VACCINE
<130> 050989.0204.02pc00
<150> US 60/746,611
   <151> 2006-05-05
<150> US 60/747,903
   <151> 2006-05-22
<160> 163
<170> PatentIn version 3.3
<210> 1
   <211> 16
   <212> PRT
   <213> Homo sapiens
<400> 1
   Met Ala Ser Gln Lys Arg Pro Ser Gln Arg His Gly Ser Lys Tyr Leu
   1               5                   10                  15
<210> 2
   <211> 16
   <212> PRT
   <213> Homo sapiens
<400> 2
   Lys Arg Pro Ser Gln Arg His Gly Ser Lys Tyr Leu Ala Thr Ala Ser
   1               5                   10                  15
<210> 3
   <211> 16
   <212> PRT
   <213> Homo sapiens
<400> 3
   Gln Arg His Gly Ser Lys Tyr Leu Ala Thr Ala Ser Thr Met Asp His
   1               5                   10                  15
<210> 4
   <211> 16
   <212> PRT
   <213> Homo sapiens
<400> 4
   Ser Lys Tyr Leu Ala Thr Ala Ser Thr Met Asp His Ala Arg His Gly
   1               5                   10                  15
<210> 5
   <211> 16
   <212> PRT
   <213> Homo sapiens
<400> 5
   Ala Thr Ala Ser Thr Met Asp His Ala Arg His Gly Phe Leu Pro Arg
   1               5                   10                  15
<210> 6
   <211> 16
   <212> PRT
   <213> Homo sapiens
<400> 6
   Thr Met Asp His Ala Arg His Gly Phe Leu Pro Arg His Arg Asp Thr
   1               5                   10                  15
<210> 7
   <211> 16
   <212> PRT
   <213> Homo sapiens
<400> 7
   Ala Arg His Gly Phe Leu Pro Arg His Arg Asp Thr Gly Ile Leu Asp
   1               5                   10                  15
<210> 8
   <211> 16
   <212> PRT
   <213> Homo sapiens
<400> 8
   Phe Leu Pro Arg His Arg Asp Thr Gly Ile Leu Asp Ser Ile Gly Arg
   1               5                   10                  15
<210> 9
   <211> 16
   <212> PRT
   <213> Homo sapiens
<400> 9
   His Arg Asp Thr Gly Ile Leu Asp Ser Ile Gly Arg Phe Phe Gly Gly
   1               5                   10                  15
<210> 10
   <211> 16
   <212> PRT
   <213> Homo sapiens
<400> 10
   Gly Ile Leu Asp Ser Ile Gly Arg Phe Phe Gly Gly Asp Arg Gly Ala
   1               5                   10                  15
<210> 11
   <211> 16
   <212> PRT
   <213> Homo sapiens
<400> 11
   Ser Ile Gly Arg Phe Phe Gly Gly Asp Arg Gly Ala Pro Lys Arg Gly
   1               5                   10                  15
<210> 12
   <211> 16
   <212> PRT
   <213> Homo sapiens
<400> 12
   Phe Phe Gly Gly Asp Arg Gly Ala Pro Lys Arg Gly Ser Gly Lys Val
   1               5                   10                  15
<210> 13
   <211> 16
   <212> PRT
   <213> Homo sapiens
<400> 13
   Asp Arg Gly Ala Pro Lys Arg Gly Ser Gly Lys Val Pro Trp Leu Lys
   1 5 10 15
<210> 14
   <211> 16
   <212> PRT
   <213> Homo sapiens
<400> 14
   Pro Lys Arg Gly Ser Gly Lys Val Pro Trp Leu Lys Pro Gly Arg Ser
   1 5 10 15
<210> 15
   <211> 16
   <212> PRT
   <213> Homo sapiens
<400> 15
   Ser Gly Lys Val Pro Trp Leu Lys Pro Gly Arg Ser Pro Leu Pro Ser
   1 5 10 15
<210> 16
   <211> 16
   <212> PRT
   <213> Homo sapiens
<400> 16
   Pro Trp Leu Lys Pro Gly Arg Ser Pro Leu Pro Ser His Ala Arg Ser
   1 5 10 15
<210> 17
   <211> 16
   <212> PRT
   <213> Homo sapiens
<400> 17
   Pro Gly Arg Ser Pro Leu Pro Ser His Ala Arg Ser Gln Pro Gly Leu
   1 5 10 15
<210> 18
   <211> 16
   <212> PRT
   <213> Homo sapiens
<400> 18
   Pro Leu Pro Ser His Ala Arg Ser Gln Pro Gly Leu Cys Asn Met Tyr
   1 5 10 15
<210> 19
   <211> 16
   <212> PRT
   <213> Homo sapiens
<400> 19
   His Ala Arg Ser Gln Pro Gly Leu Cys Asn Met Tyr Lys Asp Ser His
   1 5 10 15
<210> 20
   <211> 16
   <212> PRT
   <213> Homo sapiens
<400> 20
   Gln Pro Gly Leu Cys Asn Met Tyr Lys Asp Ser His His Pro Ala Arg
   1 5 10 15
<210> 21
   <211> 16
   <212> PRT
   <213> Homo sapiens
<400> 21
   Cys Asn Met Tyr Lys Asp Ser His His Pro Ala Arg Thr Ala His Tyr
   1 5 10 15
<210> 22
   <211> 16
   <212> PRT
   <213> Homo sapiens
<400> 22
   Lys Asp Ser His His Pro Ala Arg Thr Ala His Tyr Gly Ser Leu Pro
   1 5 10 15
<210> 23
   <211> 16
   <212> PRT
   <213> Homo sapiens
<400> 23
   His Pro Ala Arg Thr Ala His Tyr Gly Ser Leu Pro Gln Lys Ser His
   1 5 10 15
<210> 24
   <211> 16
   <212> PRT
   <213> Homo sapiens
<400> 24
   Thr Ala His Tyr Gly Ser Leu Pro Gln Lys Ser His Gly Arg Thr Gln
   1               5                   10                  15
<210> 25
   <211> 16
   <212> PRT
   <213> Homo sapiens
<400> 25
   Gly Ser Leu Pro Gln Lys Ser His Gly Arg Thr Gln Asp Glu Asn Pro
   1               5                   10                  15
<210> 26
   <211> 16
   <212> PRT
   <213> Homo sapiens
<400> 26
   Gln Lys Ser His Gly Arg Thr Gln Asp Glu Asn Pro Val Val His Phe
   1               5                   10                  15
<210> 27
   <211> 16
   <212> PRT
   <213> Homo sapiens
<400> 27
   Gly Arg Thr Gln Asp Glu Asn Pro Val Val His Phe Phe Lys Asn Ile
   1               5                   10                  15
<210> 28
   <211> 16
   <212> PRT
   <213> Homo sapiens
<400> 28
   Asp Glu Asn Pro Val Val His Phe Phe Lys Asn Ile Val Thr Pro Arg
   1               5                   10                  15
<210> 29
   <211> 16
   <212> PRT
   <213> Homo sapiens
<400> 29
   Val Val His Phe Phe Lys Asn Ile Val Thr Pro Arg Thr Pro Pro Pro
   1 5 10 15
<210> 30
   <211> 16
   <212> PRT
   <213> Homo sapiens
<400> 30
   Phe Lys Asn Ile Val Thr Pro Arg Thr Pro Pro Pro Ser Gln Gly Lys
   1 5 10 15
<210> 31
   <211> 15
   <212> PRT
   <213> Homo sapiens
<400> 31
   Val Thr Pro Arg Thr Pro Pro Pro Ser Gln Gly Gly Ala Glu Gly
   1 5 10 15
<210> 32
   <211> 15
   <212> PRT
   <213> Homo sapiens
<400> 32
   Thr Pro Pro Pro Ser Gln Gly Gly Ala Glu Gly Gln Arg Pro Gly
   1 5 10 15
<210> 33
   <211> 15
   <212> PRT
   <213> Homo sapiens
<400> 33
   Ser Gln Gly Gly Ala Glu Gly Gln Arg Pro Gly Phe Gly Tyr Gly
   1 5 10 15
<210> 34
   <211> 15
   <212> PRT
   <213> Homo sapiens
<400> 34
   Ala Glu Gly Gln Arg Pro Gly Phe Gly Tyr Gly Gly Arg Ala Ser
   1 5 10 15
<210> 35
   <211> 15
   <212> PRT
   <213> Homo sapiens
<400> 35
   Arg Pro Gly Phe Gly Tyr Gly Gly Arg Ala Ser Asp Tyr Lys Ser
   1 5 10 15
<210> 36
   <211> 15
   <212> PRT
   <213> Homo sapiens
<400> 36
   Gly Tyr Gly Gly Arg Ala Ser Asp Tyr Lys Ser Ala His Lys Gly
   1 5 10 15
<210> 37
   <211> 15
   <212> PRT
   <213> Homo sapiens
<400> 37
   Arg Ala Ser Asp Tyr Lys Ser Ala His Lys Gly Phe Lys Gly Val
   1 5 10 15
<210> 38
   <211> 15
   <212> PRT
   <213> Homo sapiens
<400> 38
   Tyr Lys Ser Ala His Lys Gly Phe Lys Gly Val Asp Ala Gln Gly
   1 5 10 15
<210> 39
   <211> 15
   <212> PRT
   <213> Homo sapiens
<400> 39
   His Lys Gly Phe Lys Gly Val Asp Ala Gln Gly Thr Leu Ser Lys
   1 5 10 15
<210> 40
   <211> 15
   <212> PRT
   <213> Homo sapiens
<400> 40
   Lys Gly Val Asp Ala Gln Gly Thr Leu Ser Lys Ile Phe Lys Leu
   1 5 10 15
<210> 41
   <211> 15
   <212> PRT
   <213> Homo sapiens
<400> 41
   Ala Gln Gly Thr Leu Ser Lys Ile Phe Lys Leu Gly Gly Arg Asp
   1 5 10 15
<210> 42
   <211> 15
   <212> PRT
   <213> Homo sapiens
<400> 42
   Leu Ser Lys Ile Phe Lys Leu Gly Gly Arg Asp Ser Arg Ser Gly
   1 5 10 15
<210> 43
   <211> 15
   <212> PRT
   <213> Homo sapiens
<400> 43
   Phe Lys Leu Gly Gly Arg Asp Ser Arg Ser Gly Ser Pro Met Ala
   1 5 10 15
<210> 44
   <211> 13
   <212> PRT
   <213> Homo sapiens
<400> 44
   Gly Arg Asp Ser Arg Ser Gly Ser Pro Met Ala Arg Arg
   1 5 10
<210> 45
   <211> 16
   <212> PRT
   <213> Homo sapiens
<400> 45
   Met Gly Leu Leu Glu Cys Cys Ala Arg Cys Leu Val Gly Ala Pro Phe
   1 5 10 15
<210> 46
   <211> 16
   <212> PRT
   <213> Homo sapiens
<400> 46
   Glu Cys Cys Ala Arg Cys Leu Val Gly Ala Pro Phe Ala Ser Leu Val
   1 5 10 15
<210> 47
   <211> 16
   <212> PRT
   <213> Homo sapiens
<400> 47
   Arg Cys Leu Val Gly Ala Pro Phe Ala Ser Leu Val Ala Thr Gly Leu
   1 5 10 15
<210> 48
   <211> 16
   <212> PRT
   <213> Homo sapiens
<400> 48
   Gly Ala Pro Phe Ala Ser Leu Val Ala Thr Gly Leu Cys Phe Phe Gly
   1 5 10 15
<210> 49
   <211> 16
   <212> PRT
   <213> Homo sapiens
<400> 49
   Ala Ser Leu Val Ala Thr Gly Leu Cys Phe Phe Gly Val Ala Leu Phe
   1 5 10 15
<210> 50
   <211> 16
   <212> PRT
   <213> Homo sapiens
<400> 50
   Ala Thr Gly Leu Cys Phe Phe Gly Val Ala Leu Phe Cys Gly Cys Gly
   1 5 10 15
<210> 51
   <211> 16
   <212> PRT
   <213> Homo sapiens
<400> 51
   Cys Phe Phe Gly Val Ala Leu Phe Cys Gly Cys Gly His Glu Ala Leu
   1 5 10 15
<210> 52
   <211> 16
   <212> PRT
   <213> Homo sapiens
<400> 52
   Val Ala Leu Phe Cys Gly Cys Gly His Glu Ala Leu Thr Gly Thr Glu
   1               5                   10                  15
<210> 53
   <211> 16
   <212> PRT
   <213> Homo sapiens
<400> 53
   Cys Gly Cys Gly His Glu Ala Leu Thr Gly Thr Glu Lys Leu Ile Glu
   1               5                   10                  15
<210> 54
   <211> 16
   <212> PRT
   <213> Homo sapiens
<400> 54
   His Glu Ala Leu Thr Gly Thr Glu Lys Leu Ile Glu Thr Tyr Phe Ser
   1               5                   10                  15
<210> 55
   <211> 16
   <212> PRT
   <213> Homo sapiens
<400> 55
   Thr Gly Thr Glu Lys Leu Ile Glu Thr Tyr Phe Ser Lys Asn Tyr Gln
   1               5                   10                  15
<210> 56
   <211> 16
   <212> PRT
   <213> Homo sapiens
<400> 56
   Lys Leu Ile Glu Thr Tyr Phe Ser Lys Asn Tyr Gln Asp Tyr Glu Tyr
   1               5                   10                  15
<210> 57
   <211> 16
   <212> PRT
   <213> Homo sapiens
<400> 57
   Thr Tyr Phe Ser Lys Asn Tyr Gln Asp Tyr Glu Tyr Leu Ile Asn Val
   1 5 10 15
<210> 58
   <211> 16
   <212> PRT
   <213> Homo sapiens
<400> 58
   Lys Asn Tyr Gln Asp Tyr Glu Tyr Leu Ile Asn Val Ile His Ala Phe
   1 5 10 15
<210> 59
   <211> 16
   <212> PRT
   <213> Homo sapiens
<400> 59
   Asp Tyr Glu Tyr Leu Ile Asn Val Ile His Ala Phe Gln Tyr Val Ile
   1 5 10 15
<210> 60
   <211> 16
   <212> PRT
   <213> Homo sapiens
<400> 60
   Leu Ile Asn Val Ile His Ala Phe Gln Tyr Val Ile Tyr Gly Thr Ala
   1 5 10 15
<210> 61
   <211> 16
   <212> PRT
   <213> Homo sapiens
<400> 61
   Ile His Ala Phe Gln Tyr Val Ile Tyr Gly Thr Ala Ser Phe Phe Phe
   1 5 10 15
<210> 62
   <211> 16
   <212> PRT
   <213> Homo sapiens
<400> 62
   Gln Tyr Val Ile Tyr Gly Thr Ala Ser Phe Phe Phe Leu Tyr Gly Ala
   1 5 10 15
<210> 63
   <211> 16
   <212> PRT
   <213> Homo sapiens
<400> 63
   Tyr Gly Thr Ala Ser Phe Phe Phe Leu Tyr Gly Ala Leu Leu Leu Ala
   1 5 10 15
<210> 64
   <211> 16
   <212> PRT
   <213> Homo sapiens
<400> 64
   Ser Phe Phe Phe Leu Tyr Gly Ala Leu Leu Leu Ala Glu Gly Phe Tyr
   1 5 10 15
<210> 65
   <211> 16
   <212> PRT
   <213> Homo sapiens
<400> 65
   Leu Tyr Gly Ala Leu Leu Leu Ala Glu Gly Phe Tyr Thr Thr Gly Ala
   1 5 10 15
<210> 66
   <211> 16
   <212> PRT
   <213> Homo sapiens
<400> 66
   Leu Leu Leu Ala Glu Gly Phe Tyr Thr Thr Gly Ala Val Arg Gln Ile
   1 5 10 15
<210> 67
   <211> 16
   <212> PRT
   <213> Homo sapiens
<400> 67
   Glu Gly Phe Tyr Thr Thr Gly Ala Val Arg Gln Ile Phe Gly Asp Tyr
   1 5 10 15
<210> 68
   <211> 16
   <212> PRT
   <213> Homo sapiens
<400> 68
   Thr Thr Gly Ala Val Arg Gln Ile Phe Gly Asp Tyr Lys Thr Thr Ile
   1 5 10 15
<210> 69
   <211> 16
   <212> PRT
   <213> Homo sapiens
<400> 69
   Val Arg Gln Ile Phe Gly Asp Tyr Lys Thr Thr Ile Cys Gly Lys Gly
   1 5 10 15
<210> 70
   <211> 16
   <212> PRT
   <213> Homo sapiens
<400> 70
   Phe Gly Asp Tyr Lys Thr Thr Ile Cys Gly Lys Gly Leu Ser Ala Thr
   1 5 10 15
<210> 71
   <211> 16
   <212> PRT
   <213> Homo sapiens
<400> 71
   Lys Thr Thr Ile Cys Gly Lys Gly Leu Ser Ala Thr Val Thr Gly Gly
   1 5 10 15
<210> 72
   <211> 16
   <212> PRT
   <213> Homo sapiens
<400> 72
   Cys Gly Lys Gly Leu Ser Ala Thr Val Thr Gly Gly Gln Lys Gly Arg
   1 5 10 15
<210> 73
   <211> 16
   <212> PRT
   <213> Homo sapiens
<400> 73
   Leu Ser Ala Thr Val Thr Gly Gly Gln Lys Gly Arg Gly Ser Arg Gly
   1 5 10 15
<210> 74
   <211> 16
   <212> PRT
   <213> Homo sapiens
<400> 74
   Val Thr Gly Gly Gln Lys Gly Arg Gly Ser Arg Gly Gln His Gln Ala
   1 5 10 15
<210> 75
   <211> 16
   <212> PRT
   <213> Homo sapiens
<400> 75
   Gln Lys Gly Arg Gly Ser Arg Gly Gln His Gln Ala His Ser Leu Glu
   1 5 10 15
<210> 76
   <211> 16
   <212> PRT
   <213> Homo sapiens
<400> 76
   Gly Ser Arg Gly Gln His Gln Ala His Ser Leu Glu Arg Val Cys His
   1 5 10 15
<210> 77
   <211> 16
   <212> PRT
   <213> Homo sapiens
<400> 77
   Gln His Gln Ala His Ser Leu Glu Arg Val Cys His Cys Leu Gly Lys
   1 5 10 15
<210> 78
   <211> 16
   <212> PRT
   <213> Homo sapiens
<400> 78
   His Ser Leu Glu Arg Val Cys His Cys Leu Gly Lys Trp Leu Gly His
   1 5 10 15
<210> 79
   <211> 16
   <212> PRT
   <213> Homo sapiens
<400> 79
   Arg Val Cys His Cys Leu Gly Lys Trp Leu Gly His Pro Asp Lys Phe
   1 5 10 15
<210> 80
   <211> 16
   <212> PRT
   <213> Homo sapiens
<400> 80
   Cys Leu Gly Lys Trp Leu Gly His Pro Asp Lys Phe Val Gly Ile Thr
   1 5 10 15
<210> 81
   <211> 16
   <212> PRT
   <213> Homo sapiens
<400> 81
   Trp Leu Gly His Pro Asp Lys Phe Val Gly Ile Thr Tyr Ala Leu Thr
   1 5 10 15
<210> 82
   <211> 16
   <212> PRT
   <213> Homo sapiens
<400> 82
   Pro Asp Lys Phe Val Gly Ile Thr Tyr Ala Leu Thr Val Val Trp Leu
   1 5 10 15
<210> 83
   <211> 16
   <212> PRT
   <213> Homo sapiens
<400> 83
   Val Gly Ile Thr Tyr Ala Leu Thr Val Val Trp Leu Leu Val Phe Ala
   1 5 10 15
<210> 84
   <211> 16
   <212> PRT
   <213> Homo sapiens
<400> 84
   Tyr Ala Leu Thr Val Val Trp Leu Leu Val Phe Ala Cys Ser Ala Val
   1 5 10 15
<210> 85
   <211> 16
   <212> PRT
   <213> Homo sapiens
<400> 85
   Val Val Trp Leu Leu Val Phe Ala Cys Ser Ala Val Pro Val Tyr Ile
   1 5 10 15
<210> 86
   <211> 16
   <212> PRT
   <213> Homo sapiens
<400> 86
   Leu Val Phe Ala Cys Ser Ala Val Pro Val Tyr Ile Tyr Phe Asn Thr
   1 5 10 15
<210> 87
   <211> 16
   <212> PRT
   <213> Homo sapiens
<400> 87
   Cys Ser Ala Val Pro Val Tyr Ile Tyr Phe Asn Thr Trp Thr Thr Cys
   1 5 10 15
<210> 88
   <211> 16
   <212> PRT
   <213> Homo sapiens
<400> 88
   Pro Val Tyr Ile Tyr Phe Asn Thr Trp Thr Thr Cys Gln Ser Ile Ala
   1 5 10 15
<210> 89
   <211> 16
   <212> PRT
   <213> Homo sapiens
<400> 89
   Tyr Phe Asn Thr Trp Thr Thr Cys Gln Ser Ile Ala Phe Pro Ser Lys
   1 5 10 15
<210> 90
   <211> 16
   <212> PRT
   <213> Homo sapiens
<400> 90
   Trp Thr Thr Cys Gln Ser Ile Ala Phe Pro Ser Lys Thr Ser Ala Ser
<210> 91
   <211> 16
   <212> PRT
   <213> Homo sapiens
<400> 91
   Gln Ser Ile Ala Phe Pro Ser Lys Thr Ser Ala Ser Ile Gly Ser Leu
   1               5                   10                  15
<210> 92
   <211> 16
   <212> PRT
   <213> Homo sapiens
<400> 92
   Phe Pro Ser Lys Thr Ser Ala Ser Ile Gly Ser Leu Cys Ala Asp Ala
   1               5                   10                  15
<210> 93
   <211> 16
   <212> PRT
   <213> Homo sapiens
<400> 93
   Thr Ser Ala Ser Ile Gly Ser Leu Cys Ala Asp Ala Arg Met Tyr Gly
   1               5                   10                  15
<210> 94
   <211> 16
   <212> PRT
   <213> Homo sapiens
<400> 94
   Ile Gly Ser Leu Cys Ala Asp Ala Arg Met Tyr Gly Val Leu Pro Trp
   1               5                   10                  15
<210> 95
   <211> 16
   <212> PRT
   <213> Homo sapiens
<400> 95
   Cys Ala Asp Ala Arg Met Tyr Gly Val Leu Pro Trp Asn Ala Phe Pro
   1               5                   10                  15
<210> 96
   <211> 16
   <212> PRT
   <213> Homo sapiens
<400> 96
   Arg Met Tyr Gly Val Leu Pro Trp Asn Ala Phe Pro Gly Lys Val Cys
   1 5 10 15
<210> 97
   <211> 16
   <212> PRT
   <213> Homo sapiens
<400> 97
   Val Leu Pro Trp Asn Ala Phe Pro Gly Lys Val Cys Gly Ser Asn Leu
   1 5 10 15
<210> 98
   <211> 16
   <212> PRT
   <213> Homo sapiens
<400> 98
   Asn Ala Phe Pro Gly Lys Val Cys Gly Ser Asn Leu Leu Ser Ile Cys
   1 5 10 15
<210> 99
   <211> 16
   <212> PRT
   <213> Homo sapiens
<400> 99
   Gly Lys Val Cys Gly Ser Asn Leu Leu Ser Ile Cys Lys Thr Ala Glu
   1 5 10 15
<210> 100
   <211> 16
   <212> PRT
   <213> Homo sapiens
<400> 100
   Gly Ser Asn Leu Leu Ser Ile Cys Lys Thr Ala Glu Phe Gln Met Thr
   1 5 10 15
<210> 101
   <211> 16
   <212> PRT
   <213> Homo sapiens
<400> 101
   Leu Ser Ile Cys Lys Thr Ala Glu Phe Gln Met Thr Phe His Leu Phe
   1 5 10 15
<210> 102
   <211> 16
   <212> PRT
   <213> Homo sapiens
<400> 102
   Lys Thr Ala Glu Phe Gln Met Thr Phe His Leu Phe Ile Ala Ala Phe
   1 5 10 15
<210> 103
   <211> 16
   <212> PRT
   <213> Homo sapiens
<400> 103
   Phe Gln Met Thr Phe His Leu Phe Ile Ala Ala Phe Val Gly Ala Ala
   1 5 10 15
<210> 104
   <211> 16
   <212> PRT
   <213> Homo sapiens
<400> 104
   Phe His Leu Phe Ile Ala Ala Phe Val Gly Ala Ala Ala Thr Leu Val
   1 5 10 15
<210> 105
   <211> 16
   <212> PRT
   <213> Homo sapiens
<400> 105
   Ile Ala Ala Phe Val Gly Ala Ala Ala Thr Leu Val Ser Leu Leu Thr
   1 5 10 15
<210> 106
   <211> 16
   <212> PRT
   <213> Homo sapiens
<400> 106
   Val Gly Ala Ala Ala Thr Leu Val Ser Leu Leu Thr Phe Met Ile Ala
   1 5 10 15
<210> 107
   <211> 16
   <212> PRT
   <213> Homo sapiens
<400> 107
   Ala Thr Leu Val Ser Leu Leu Thr Phe Met Ile Ala Ala Thr Tyr Asn
   1 5 10 15
<210> 108
   <211> 16
   <212> PRT
   <213> Homo sapiens
<400> 108
   Ser Leu Leu Thr Phe Met Ile Ala Ala Thr Tyr Asn Phe Ala Val Leu
   1 5 10 15
<210> 109
   <211> 16
   <212> PRT
   <213> Homo sapiens
<400> 109
   Phe Met Ile Ala Ala Thr Tyr Asn Phe Ala Val Leu Lys Leu Met Gly
   1 5 10 15
<210> 110
   <211> 16
   <212> PRT
   <213> Homo sapiens
<400> 110
   Ala Thr Tyr Asn Phe Ala Val Leu Lys Leu Met Gly Arg Gly Thr Lys
   1 5 10 15
<210> 111
   <211> 13
   <212> PRT
   <213> Homo sapiens
<400> 111
   Phe Ala Val Leu Lys Leu Met Gly Arg Gly Thr Lys Phe
   1 5 10
<210> 112
   <211> 16
   <212> PRT
   <213> Homo sapiens
<400> 112
   Gly Gln Phe Arg Val Ile Gly Pro Arg His Pro Ile Arg Ala Leu Val
   1 5 10 15
<210> 113
   <211> 16
   <212> PRT
   <213> Homo sapiens
<400> 113
   Val Ile Gly Pro Arg His Pro Ile Arg Ala Leu Val Gly Asp Glu Val
   1 5 10 15
<210> 114
   <211> 16
   <212> PRT
   <213> Homo sapiens
<400> 114
   Arg His Pro Ile Arg Ala Leu Val Gly Asp Glu Val Glu Leu Pro Cys
   1 5 10 15
<210> 115
   <211> 16
   <212> PRT
   <213> Homo sapiens
<400> 115
   Arg Ala Leu Val Gly Asp Glu Val Glu Leu Pro Cys Arg Ile Ser Pro
   1 5 10 15
<210> 116
   <211> 16
   <212> PRT
   <213> Homo sapiens
<400> 116
   Gly Asp Glu Val Glu Leu Pro Cys Arg Ile Ser Pro Gly Lys Asn Ala
   1 5 10 15
<210> 117
   <211> 16
   <212> PRT
   <213> Homo sapiens
<400> 117
   Glu Leu Pro Cys Arg Ile Ser Pro Gly Lys Asn Ala Thr Gly Met Glu
   1 5 10 15
<210> 118
   <211> 16
   <212> PRT
   <213> Homo sapiens
<400> 118
   Arg Ile Ser Pro Gly Lys Asn Ala Thr Gly Met Glu Val Gly Trp Tyr
   1 5 10 15
<210> 119
   <211> 16
   <212> PRT
   <213> Homo sapiens
<400> 119
   Gly Lys Asn Ala Thr Asx Met Glu Val Gly Trp Tyr Arg Pro Pro Phe
   1 5 10 15
<210> 120
   <211> 16
   <212> PRT
   <213> Homo sapiens
<400> 120
   Thr Gly Met Glu Val Gly Trp Tyr Arg Pro Pro Phe Ser Arg Val Val
   1 5 10 15
<210> 121
   <211> 16
   <212> PRT
   <213> Homo sapiens
<400> 121
   Val Gly Trp Tyr Arg Pro Pro Phe Ser Arg Val Val His Leu Tyr Arg
   1 5 10 15
<210> 122
   <211> 16
   <212> PRT
   <213> Homo sapiens
<400> 122
   Arg Pro Pro Phe Ser Arg Val Val His Leu Tyr Arg Asn Gly Lys Asp
   1 5 10 15
<210> 123
   <211> 16
   <212> PRT
   <213> Homo sapiens
<400> 123
   Ser Arg Val Val His Leu Tyr Arg Asn Gly Lys Asp Gln Asp Gly Asp
   1 5 10 15
<210> 124
   <211> 16
   <212> PRT
   <213> Homo sapiens
<400> 124
   His Leu Tyr Arg Asn Gly Lys Asp Gln Asp Gly Asp Gln Ala Pro Glu
   1 5 10 15
<210> 125
   <211> 16
   <212> PRT
   <213> Homo sapiens
<400> 125
   Asn Gly Lys Asp Gln Asp Gly Asp Gln Ala Pro Glu Tyr Arg Gly Arg
   1 5 10 15
<210> 126
   <211> 16
   <212> PRT
   <213> Homo sapiens
<400> 126
   Gln Asp Gly Asp Gln Ala Pro Glu Tyr Arg Gly Arg Thr Glu Leu Leu
   1 5 10 15
<210> 127
   <211> 16
   <212> PRT
   <213> Homo sapiens
<400> 127
   Gln Ala Pro Glu Tyr Arg Gly Arg Thr Glu Leu Leu Lys Asp Ala Ile
   1 5 10 15
<210> 128
   <211> 16
   <212> PRT
   <213> Homo sapiens
<400> 128
   Tyr Arg Gly Arg Thr Glu Leu Leu Lys Asp Ala Ile Gly Glu Gly Lys
   1 5 10 15
<210> 129
   <211> 16
   <212> PRT
   <213> Homo sapiens
<400> 129
   Thr Glu Leu Leu Lys Asp Ala Ile Gly Glu Gly Lys Val Thr Leu Arg
   1 5 10 15
<210> 130
   <211> 16
   <212> PRT
   <213> Homo sapiens
<400> 130
   Lys Asp Ala Ile Gly Glu Gly Lys Val Thr Leu Arg Ile Arg Asn Val
   1 5 10 15
<210> 131
   <211> 16
   <212> PRT
   <213> Homo sapiens
<400> 131
   Gly Glu Gly Lys Val Thr Leu Arg Ile Arg Asn Val Arg Phe Ser Asp
   1 5 10 15
<210> 132
   <211> 16
   <212> PRT
   <213> Homo sapiens
<400> 132
   Val Thr Leu Arg Ile Arg Asn Val Arg Phe Ser Asp Glu Gly Gly Phe
   1 5 10 15
<210> 133
   <211> 16
   <212> PRT
   <213> Homo sapiens
<400> 133
   Ile Arg Asn Val Arg Phe Ser Asp Glu Gly Gly Phe Thr Cys Phe Phe
   1 5 10 15
<210> 134
   <211> 16
   <212> PRT
   <213> Homo sapiens
<400> 134
   Arg Phe Ser Asp Glu Gly Gly Phe Thr Cys Phe Phe Arg Asp His Ser
   1 5 10 15
<210> 135
   <211> 16
   <212> PRT
   <213> Homo sapiens
<400> 135
   Glu Gly Gly Phe Thr Cys Phe Phe Arg Asp His Ser Tyr Gln Glu Glu
   1 5 10 15
<210> 136
   <211> 16
   <212> PRT
   <213> Homo sapiens
<400> 136
   Thr Cys Phe Phe Arg Asp His Ser Tyr Gln Glu Glu Ala Ala Met Glu
   1 5 10 15
<210> 137
   <211> 16
   <212> PRT
   <213> Homo sapiens
<400> 137
   Arg Asp His Ser Tyr Gln Glu Glu Ala Ala Met Glu Leu Lys Val Glu
   1 5 10 15
<210> 138
   <211> 16
   <212> PRT
   <213> Homo sapiens
<400> 138
   Tyr Gln Glu Glu Ala Ala Met Glu Leu Lys Val Glu Asp Pro Phe Tyr
   1 5 10 15
<210> 139
   <211> 16
   <212> PRT
   <213> Homo sapiens
<400> 139
   Ala Ala Met Glu Leu Lys Val Glu Asp Pro Phe Tyr Trp Val Ser Pro
   1 5 10 15
<210> 140
   <211> 16
   <212> PRT
   <213> Homo sapiens
<400> 140
   Leu Lys Val Glu Asp Pro Phe Tyr Trp Val Ser Pro Gly Val Leu Val
   1               5                   10                  15
<210> 141
   <211> 16
   <212> PRT
   <213> Homo sapiens
<400> 141
   Asp Pro Phe Tyr Trp Val Ser Pro Gly Val Leu Val Leu Leu Ala Val
   1               5                   10                  15
<210> 142
   <211> 16
   <212> PRT
   <213> Homo sapiens
<400> 142
   Trp Val Ser Pro Gly Val Leu Val Leu Leu Ala Val Leu Pro Val Leu
   1               5                   10                  15
<210> 143
   <211> 16
   <212> PRT
   <213> Homo sapiens
<400> 143
   Gly Val Leu Val Leu Leu Ala Val Leu Pro Val Leu Leu Leu Gln Ile
   1               5                   10                  15
<210> 144
   <211> 16
   <212> PRT
   <213> Homo sapiens
<400> 144
   Leu Leu Ala Val Leu Pro Val Leu Leu Leu Gln Ile Thr Val Gly Leu
   1               5                   10                  15
<210> 145
   <211> 16
   <212> PRT
   <213> Homo sapiens
<400> 145
   Leu Pro Val Leu Leu Leu Gln Ile Thr Val Gly Leu Val Phe Leu Cys
   1 5 10 15
<210> 146
   <211> 16
   <212> PRT
   <213> Homo sapiens
<400> 146
   Leu Leu Gln Ile Thr Val Gly Leu Val Phe Leu Cys Leu Gln Tyr Arg
   1 5 10 15
<210> 147
   <211> 16
   <212> PRT
   <213> Homo sapiens
<400> 147
   Thr Val Gly Leu Val Phe Leu Cys Leu Gln Tyr Arg Leu Arg Gly Lys
   1 5 10 15
<210> 148
   <211> 16
   <212> PRT
   <213> Homo sapiens
<400> 148
   Val Phe Leu Cys Leu Gln Tyr Arg Leu Arg Gly Lys Leu Arg Ala Glu
   1 5 10 15
<210> 149
   <211> 16
   <212> PRT
   <213> Homo sapiens
<400> 149
   Leu Gln Tyr Arg Leu Arg Gly Lys Leu Arg Ala Glu Ile Glu Asn Leu
   1 5 10 15
<210> 150
   <211> 16
   <212> PRT
   <213> Homo sapiens
<400> 150
   Leu Arg Gly Lys Leu Arg Ala Glu Ile Glu Asn Leu His Arg Thr Phe
   1 5 10 15
<210> 151
   <211> 16
   <212> PRT
   <213> Homo sapiens
<400> 151
   Leu Arg Ala Glu Ile Glu Asn Leu His Arg Thr Phe Asp Pro His Phe
   1 5 10 15
<210> 152
   <211> 16
   <212> PRT
   <213> Homo sapiens
<400> 152
   Ile Glu Asn Leu His Arg Thr Phe Asp Pro His Phe Leu Arg Val Pro
   1 5 10 15
<210> 153
   <211> 16
   <212> PRT
   <213> Homo sapiens
<400> 153
   His Arg Thr Phe Asp Pro His Phe Leu Arg Val Pro Cys Trp Lys Ile
   1 5 10 15
<210> 154
   <211> 16
   <212> PRT
   <213> Homo sapiens
<400> 154
   Asp Pro His Phe Leu Arg Val Pro Cys Trp Lys Ile Thr Leu Phe Val
   1 5 10 15
<210> 155
   <211> 16
   <212> PRT
   <213> Homo sapiens
<400> 155
   Leu Arg Val Pro Cys Trp Lys Ile Thr Leu Phe Val Ile Val Pro Val
   1 5 10 15
<210> 156
   <211> 16
   <212> PRT
   <213> Homo sapiens
<400> 156
   Cys Trp Lys Ile Thr Leu Phe Val Ile Val Pro Val Leu Gly Pro Leu
   1               5                   10                  15
<210> 157
   <211> 16
   <212> PRT
   <213> Homo sapiens
<400> 157
   Thr Leu Phe Val Ile Val Pro Val Leu Gly Pro Leu Val Ala Leu Ile
   1               5                   10                  15
<210> 158
   <211> 16
   <212> PRT
   <213> Homo sapiens
<400> 158
   Ile Val Pro Val Leu Gly Pro Leu Val Ala Leu Ile Ile Cys Tyr Asn
   1               5                   10                  15
<210> 159
   <211> 16
   <212> PRT
   <213> Homo sapiens
<400> 159
   Leu Gly Pro Leu Val Ala Leu Ile Ile Cys Tyr Asn Trp Leu His Arg
   1               5                   10                  15
<210> 160
   <211> 16
   <212> PRT
   <213> Homo sapiens
<400> 160
   Val Ala Leu Ile Ile Cys Tyr Asn Trp Leu His Arg Arg Leu Ala Gly
   1               5                   10                  15
<210> 161
   <211> 16
   <212> PRT
   <213> Homo sapiens
<400> 161
   Ile Cys Tyr Asn Trp Leu His Arg Arg Leu Ala Gly Gln Phe Leu Glu
   1               5                   10                  15
<210> 162
   <211> 16
   <212> PRT
   <213> Homo sapiens
<400> 162
   Trp Leu His Arg Arg Leu Ala Gly Gln Phe Leu Glu Glu Leu Arg Asn
   1 5 10 15
<210> 163
   <211> 14
   <212> PRT
   <213> Homo sapiens
<400> 163
   Arg Leu Ala Gly Gln Phe Leu Glu Glu Leu Arg Asn Pro Phe
   1 5 10